(19) **Europäisches Patentamt / European Patent Office / Office européen des brevets**

(11) **EP 2 792 745 B1**

(12) **EUROPEAN PATENT SPECIFICATION**

(45) Date of publication and mention
of the grant of the patent:
**15.11.2017 Bulletin 2017/46**

(21) Application number: **12858631.0**

(22) Date of filing: **14.12.2012**

(51) Int Cl.:
*C12N 15/09* (2006.01)    *A61K 39/00* (2006.01)
*A61K 39/12* (2006.01)    *A61K 39/245* (2006.01)
*A61K 39/39* (2006.01)    *A61P 1/16* (2006.01)
*A61P 1/18* (2006.01)    *A61P 3/10* (2006.01)
*A61P 19/02* (2006.01)    *A61P 25/00* (2006.01)
*A61P 35/00* (2006.01)    *A61P 35/02* (2006.01)
*A61P 37/00* (2006.01)    *A61P 37/08* (2006.01)
*C07K 7/08* (2006.01)    *C07K 14/77* (2006.01)
*C07K 14/82* (2006.01)

(86) International application number:
**PCT/JP2012/082573**

(87) International publication number:
**WO 2013/089252 (20.06.2013 Gazette 2013/25)**

(54) **MODIFICATION OF HELPER T CELL-INDUCING POLYPEPTIDE**

MODIFIZIERUNG EINES HELFER-T-ZELLEN-INDUZIERENDEN POLYPEPTIDS

MODIFICATION DE POLYPEPTIDE INDUISANT DES CELLULES T AUXILIAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.12.2011 JP 2011273922**

(43) Date of publication of application:
**22.10.2014 Bulletin 2014/43**

(73) Proprietor: **National University Corporation
Kochi University
Kochi-shi, Kochi 780-8520 (JP)**

(72) Inventor: **UDAKA, Keiko
Kochi-shi
Kochi 780-8520 (JP)**

(74) Representative: **J A Kemp
14 South Square
Gray's Inn
London WC1R 5JJ (GB)**

(56) References cited:
EP-A1- 2 119 778    EP-A1- 2 270 144
EP-A1- 2 423 310    WO-A1-00/22112
WO-A1-98/33511    WO-A1-2005/045027
WO-A1-2008/096831    WO-A1-2009/123188
WO-A1-2010/123065    JP-A- 2009 509 518

- **TSUBOI AKIHIRO ET AL: "Enhanced induction of human WT1-specific cytotoxic T lymphocytes with a 9-mer WT1 peptide modified at HLA-A*2402-binding residues.", CANCER IMMUNOLOGY IMMUNOTHERAPY, vol. 51, no. 11-12, December 2002 (2002-12), pages 614-620, XP002741380, ISSN: 0340-7004**

- **MAILLERE B. ET AL.: 'Fine chemical modifications at N- and C-termini enhance peptide presentation to T cells by increasing the lifespan of both free and MHC-complexed peptides.' MOL.IMMUNOL. vol. 32, no. 17-18, December 1995, pages 1377 - 85, XP002984101**

- **BRINCKERHOFF L.H. ET AL.: 'Terminal modifications inhibit proteolytic degradation of an immunogenic MART-1(27-35) peptide: implications for peptide vaccines.' INT.J.CANCER. vol. 83, no. 3, 29 October 1999, pages 326 - 334, XP002303298**

- **MARASTONI M. ET AL.: 'Peptide analogues of a subdominant epitope expressed in ebv-associated tumors: synthesis and immunological activity.' J.MED.CHEM. vol. 44, no. 14, 05 July 2001, pages 2370 - 3, XP055154673**

- **AYYOUB M. ET AL.: 'A structure-based approach to designing non-natural peptides that can activate anti-melanoma cytotoxic T cells.' J. BIOL.CHEM. vol. 274, no. 15, 09 April 1999, pages 10227 - 34, XP002198765**

- AYYOUB M. ET AL.: 'Analysis of the degradation mechanisms of MHC class I-presented tumor antigenic peptides by high performance liquid chromatography/electrospray ionization mass spectrometry: application to the design of peptidase-resistant analogs.' RAPID COMMUN.MASS SPECTROM. vol. 12, no. 9, 1998, pages 557 - 64, XP008037828

SEQUENCE LISTING

<110> National University Corporation Kochi University

<120> Modification of helper T cell inducing polypeptide

<130> 091965

<150> JP 2011-273922
<151> 2011-12-14

<160> 41

<170> PatentIn version 3.3

<210> 1
<211> 16
<212> PRT
<213> Homo sapiens

<400> 1

```
Lys Arg Tyr Phe Lys Leu Ser His Leu Gln Met His Ser Arg Lys His
1               5                   10                  15
```

<210> 2
<211> 15
<212> PRT
<213> Homo sapiens

<400> 2

```
Pro Val Leu Asp Phe Ala Pro Pro Gly Ala Ser Ala Tyr Gly Ser
1               5                   10                  15
```

<210> 3
<211> 20
<212> PRT
<213> Homo sapiens

<400> 3

```
Gly Val Leu Gln Gly Ile Thr Ser Trp Gly Ser Glu Pro Cys Ala Leu
1               5                   10                  15

Pro Glu Arg Pro
            20
```

<210> 4
<211> 16
<212> PRT
<213> Homo sapiens

<400> 4

```
        Lys Lys Leu Leu Thr Gln His Phe Val Gln Glu Asn Tyr Leu Glu Tyr
        1               5                   10                  15
```

<210> 5
<211> 13
<212> PRT
<213> Homo sapiens

<400> 5

```
        Leu Trp Trp Val Asn Asn Gln Ser Leu Pro Val Ser Pro
        1               5                   10
```

<210> 6
<211> 15
<212> PRT
<213> Homo sapiens

<400> 6

```
        Tyr Ala Cys Phe Val Ser Asn Leu Ala Thr Gly Arg Asn Asn Ser
        1               5                   10                  15
```

<210> 7
<211> 15
<212> PRT
<213> Homo sapiens

<400> 7

```
        Thr Leu Gly Glu Phe Leu Lys Leu Asp Arg Glu Arg Ala Lys Asn
        1               5                   10                  15
```

<210> 8
<211> 11
<212> PRT
<213> Homo sapiens

<400> 8

```
        Glu Ile Trp Arg Asp Ile Asp Phe Ala His Glu
        1               5                   10
```

<210> 9
<211> 9
<212> PRT
<213> Hepatitis C virus

<400> 9

```
        Ala Leu Tyr Gly Val Trp Pro Leu Leu
        1               5
```

<210> 10
<211> 20
<212> PRT

<213> Hepatitis C virus

<400> 10

```
Asp Pro Arg Arg Arg Ser Arg Asn Leu Gly Lys Val Ile Asp Thr Phe
1               5                   10                  15

Thr Cys Gly Leu
            20
```

<210> 11
<211> 20
<212> PRT
<213> Hepatitis C virus

<400> 11

```
Ser Val Asn Tyr Ala Thr Gly Asn Leu Pro Gly Cys Ser Phe Ser Ile
1               5                   10                  15

Phe Leu Leu Ala
            20
```

<210> 12
<211> 17
<212> PRT
<213> Epstein-Barr virus

<400> 12

```
Tyr Leu Gln Gln Asn Trp Trp Thr Leu Leu Val Asp Leu Leu Trp Leu
1               5                   10                  15

Leu
```

<210> 13
<211> 9
<212> PRT
<213> Homo sapiens

<400> 13

```
Ala Leu Leu Pro Ala Val Pro Ser Leu
1               5
```

<210> 14
<211> 9
<212> PRT
<213> Homo sapiens

<400> 14

```
                    Arg Val Pro Gly Val Ala Pro Thr Leu
                    1               5
```

<210> 15
<211> 9
<212> PRT
<213> Homo sapiens

<400> 15

```
                    Gly Val Phe Arg Gly Ile Gln Asp Val
                    1               5
```

<210> 16
<211> 17
<212> PRT
<213> Artificial

<220>
<223> peptide inducing Th

<400> 16

```
        Ile Ser Gln Ala Val His Ala Ala His Ala Glu Ile Asn Glu Ala Gly
        1               5                   10                  15


        Arg
```

<210> 17
<211> 15
<212> PRT
<213> Artificial

<220>
<223> peptide inducing Th

<400> 17

```
        Ile Ser Gln Ala Val His Ala Ala His Ala Glu Ile Asn Glu Ala
        1               5                   10                  15
```

<210> 18
<211> 15
<212> PRT
<213> Artificial

<220>
<223> peptide inducing Th

<220>
<221> MOD_RES
<222> (1)..(1)
<223> ACETYLATION

<400> 18

```
Ile Ser Gln Ala Val His Ala Ala His Ala Glu Ile Asn Glu Ala
1               5                   10                  15
```

<210> 19
<211> 17
<212> PRT
<213> Artificial

<220>
<223> peptide inducing Th

<220>
<221> MOD_RES
<222> (17)..(17)
<223> bAla

<400> 19

```
Ile Ser Gln Ala Val His Ala Ala His Ala Glu Ile Asn Glu Ala Gly
1               5                   10                  15

Xaa
```

<210> 20
<211> 17
<212> PRT
<213> Artificial

<220>
<223> peptide inducing Th

<400> 20

```
Ile Ser Gln Ala Val His Ala Ala His Ala Glu Ile Asn Glu Ala Pro
1               5                   10                  15

Ala
```

<210> 21
<211> 17
<212> PRT
<213> Artificial

<220>
<223> peptide inducing Th

<220>
<221> MOD_RES
<222> (17)..(17)
<223> bAla

<400> 21

```
        Ile Ser Gln Ala Val His Ala Ala His Ala Glu Ile Asn Glu Ala Pro
        1                 5                 10                15


        Xaa
```

<210> 22
<211> 17
<212> PRT
<213> Artificial

<220>
<223> peptide inducing Th

<400> 22

```
        Pro Ser Ile Ser Gln Ala Val His Ala Ala His Ala Glu Ile Asn Glu
        1                 5                 10                15


        Ala
```

<210> 23
<211> 19
<212> PRT
<213> Artificial

<220>
<223> peptide inducing Th

<220>
<221> MOD_RES
<222> (19)..(19)
<223> bAla

<400> 23

```
        Pro Ser Ile Ser Gln Ala Val His Ala Ala His Ala Glu Ile Asn Glu
        1                 5                 10                15


        Ala Pro Xaa
```

<210> 24
<211> 16
<212> PRT
<213> Artificial

<220>
<223> peptide inducing Th

<400> 24

```
        Ile Ser Gln Ala Val His Ala Ala His Ala Glu Ile Asn Glu Ala Arg
        1                 5                 10                15
```

<210> 25
<211> 18

<212> PRT
<213> Artificial

<220>
<223> peptide inducing Th

<220>
<221> MOD_RES
<222> (2)..(2)
<223> Lys is biotinylated

<400> 25

```
Ser Lys Ile Ser Gln Ala Val His Ala Ala His Ala Glu Ile Asn Glu
1               5                   10                  15

Ala Arg
```

<210> 26
<211> 20
<212> PRT
<213> Artificial

<220>
<223> peptide inducing Th

<220>
<221> MOD_RES
<222> (19)..(19)
<223> Lys is biotinylated

<400> 26

```
Ser Lys Ile Ser Gln Ala Val His Ala Ala His Ala Glu Ile Asn Glu
1               5                   10                  15

Ala Arg Lys Gly
            20
```

<210> 27
<211> 19
<212> PRT
<213> Artificial

<220>
<223> peptide inducing Th

<220>
<221> MOD_RES
<222> (2)..(2)
<223> Lys is biotinylated

<220>
<221> MOD_RES
<222> (19)..(19)
<223> Lys is biotinylated

<400> 27

```
Ser Lys Ile Ser Gln Ala Val His Ala Ala His Ala Glu Ile Asn Glu
1               5                   10                  15

Ala Arg Lys
```

<210> 28
<211> 19
<212> PRT
<213> Artificial

<220>
<223> peptide inducing Th

<220>
<221> MOD_RES
<222> (2)..(2)
<223> Lys is biotinylated

<220>
<221> MOD_RES
<222> (18)..(18)
<223> Lys is biotinylated

<400> 28

```
Ser Lys Ile Ser Gln Ala Val His Ala Ala His Ala Glu Ile Asn Glu
1               5                   10                  15

Ala Lys Gly
```

<210> 29
<211> 20
<212> PRT
<213> Artificial

<220>
<223> peptide inducing Th

<220>
<221> MOD_RES
<222> (2)..(2)
<223> Lys is biotinylated

<220>
<221> MOD_RES
<222> (19)..(19)
<223> Lys is biotinylated

<400> 29

```
Ser Lys Ile Ser Gln Ala Val His Ala Ala His Ala Glu Ile Asn Glu
1               5                   10                  15
```

```
Ala Arg Lys Gly
            20
```

<210> 30
<211> 21
<212> PRT
<213> Artificial

<220>
<223> peptide inducing Th

<220>
<221> MOD_RES
<222> (2)..(2)
<223> Lys is biotinylated

<220>
<221> MOD_RES
<222> (19)..(19)
<223> Lys is biotinylated

<220>
<221> MOD_RES
<222> (21)..(21)
<223> bAla

<400> 30

```
Ser Lys Ile Ser Gln Ala Val His Ala Ala His Ala Glu Ile Asn Glu
1               5                   10                  15
```

```
Ala Arg Lys Pro Xaa
            20
```

<210> 31
<211> 21
<212> PRT
<213> Artificial

<220>
<223> peptide inducing Th

<220>
<221> MOD_RES
<222> (3)..(3)
<223> Lys is biotinylated

<220>
<221> MOD_RES
<222> (19)..(19)
<223> Lys is biotinylated

<220>

<221> MOD_RES
<222> (21)..(21)
<223> bAla

<400> 31

```
Pro Ser Lys Ile Ser Gln Ala Val His Ala Ala His Ala Glu Ile Asn
1               5                   10                  15

                            Glu Ala Lys Pro Xaa
                                         20
```

<210> 32
<211> 22
<212> PRT
<213> Artificial

<220>
<223> peptide inducing Th

<220>
<221> MOD_RES
<222> (3)..(3)
<223> Lys is biotinylated

<220>
<221> MOD_RES
<222> (20)..(20)
<223> Lys is biotinylated

<220>
<221> MOD_RES
<222> (22)..(22)
<223> bAla

<400> 32

```
Pro Ser Lys Ile Ser Gln Ala Val His Ala Ala His Ala Glu Ile Asn
1               5                   10                  15


Glu Ala Arg Lys Pro Xaa
         20
```

<210> 33
<211> 18
<212> PRT
<213> Artificial

<220>
<223> peptide inducing Th

<400> 33

Ser Lys Ile Ser Gln Ala Val His Ala Ala His Ala Glu Ile Asn Glu
1               5                   10                  15

Ala Arg

<210> 34
<211> 19
<212> PRT
<213> Artificial

<220>
<223> peptide inducing Th

<400> 34

Ser Lys Ile Ser Gln Ala Val His Ala Ala His Ala Glu Ile Asn Glu
1               5                   10                  15

Ala Arg Lys

<210> 35
<211> 15
<212> PRT
<213> Artificial

<220>
<223> peptide inducing Th

<400> 35

Asn Glu Arg Ala Asp Leu Ile Ala Tyr Leu Lys Gln Ala Thr Lys
1               5                   10                  15

<210> 36
<211> 21
<212> PRT
<213> Artificial

<220>
<223> peptide inducing Th

<220>
<221> MOD_RES
<222> (3)..(3)
<223> Lys is biotinylated

<220>
<221> MOD_RES
<222> (19)..(19)
<223> Lys is biotinylated

<220>
<221> MOD_RES
<222> (21)..(21)
<223> bAla

<400> 36

```
Pro Ser Lys Asn Glu Arg Ala Asp Leu Ile Ala Tyr Leu Lys Gln Ala
1               5                   10                  15

Thr Lys Lys Pro Xaa
                20
```

<210> 37
<211> 21
<212> PRT
<213> Artificial

<220>
<223> peptide inducing Th

<220>
<221> MOD_RES
<222> (3)..(3)
<223> Lys is biotinylated

<220>
<221> MOD_RES
<222> (19)..(19)
<223> Lys is biotinylated

<220>
<221> MOD_RES
<222> (21)..(21)
<223> bAla

<400> 37

```
Pro Ser Lys Pro Val Leu Asp Phe Ala Pro Pro Gly Ala Ser Ala Tyr
1               5                   10                  15

Gly Ser Lys Pro Xaa
                20
```

<210> 38
<211> 22
<212> PRT
<213> Artificial

<220>
<223> peptide inducing Th

<220>
<221> MOD_RES
<222> (3)..(3)
<223> Lys is biotinylated

<220>
<221> MOD_RES
<222> (20)..(20)
<223> Lys is biotinylated

<220>
<221> MOD_RES
<222> (22)..(22)
<223> bAla

<400> 38

```
Pro Ser Lys Lys Arg Tyr Phe Lys Leu Ser His Leu Gln Met His Ser
1               5                   10                  15

Arg Lys His Lys Pro Xaa
                    20
```

<210> 39
<211> 15
<212> PRT
<213> Artificial

<220>
<223> peptide inducing Th

<400> 39

```
Leu Glu Asp Ala Arg Arg Leu Lys Ala Ile Tyr Glu Lys Lys Lys
1               5                   10                  15
```

<210> 40
<211> 20
<212> PRT
<213> Artificial

<220>
<223> peptide inducing Th

<220>
<221> MOD_RES
<222> (20)..(20)
<223> bAla

<400> 40

```
Pro Ser Lys Arg Tyr Phe Lys Leu Ser His Leu Gln Met His Ser Arg
1               5                   10                  15

Lys His Pro Xaa
            20
```

<210> 41
<211> 18
<212> PRT
<213> Artificial

<220>
<223> peptide inducing Th

```
<220>
<221> MOD_RES
<222> (3)..(3)
<223> Lys is biotinylated

<220>
<221> MOD_RES
<222> (16)..(16)
<223> Lys is biotinylated

<220>
<221> MOD_RES
<222> (18)..(18)
<223> bAla

<400> 41
```

```
Pro Ser Lys Arg Glu Phe Lys Leu Ser Lys Val Trp Arg Asp Gln Lys
1               5               10                  15


Pro Xaa
```

**Description**

Technical Field

The present invention relates to a modified molecule of helper T cell (Th)-inducing polypeptide and an antitumor agent containing the modified molecule and the like.

Background Art

In the immunity against tumor, CD8[+]cytotoxic T lymphocyte (CTL) plays a key role as an effector to kill tumor cells. In addition, CD4[+] helper T (Th) cell, particularly Th1 type Th cell, has been reported to be essential for the induction of efficient antitumor immune activity (non-patent documents 1 - 4). There are various reports that have been made heretofore on the role of Th cell in antitumor immunity, and induction of differentiation and growth of CTL (non-patent document 5), rendering antigen presenting cell (APC) capable of direct activation of CTL, and activation of CTL by cross presentation of antigen to APC (non-patent documents 6, 7), anti-apoptosis effect on CTL and APC (non-patent documents 8, 9), function to help growth of memory CD8 T cells and preservation and expansion of peripheral pool (non-patent documents 10, 11), induction of infiltration of CTL into tumor tissues (non-patent documents 12, 13) and the like are known.

Many studies have been made as to a method utilizing antitumor immunity to treat a malignant tumor. For example, in an immunotherapy using HLA (human leukocyte antigen; the below-mentioned MHC for human is to be referred to as HLA) class I binding tumor antigen-specific polypeptide that induces CTL, the polypeptide is administered singly, or together with non-methylated deoxy CpG, Toll-like receptor ligand or adjuvant such as Flt3. However, by such method, CTL may grow but is difficult to activate to a level sufficient as an effector, and the clinical response is limited. As a new antitumor immunotherapy, an immunotherapy using an HLA class I binding tumor antigen polypeptide that induces CTL and an HLA class II binding tumor antigen polypeptide that induces Th cell is also considered. However, such method does not necessarily activate tumor antigen-specific Th cell but, instead, may induce regulatory T cells ($T_{reg}$) that suppress immunity against the tumor antigen, as a result of which the antitumor immunity may be suppressed (non-patent document 14). Particularly in antitumor immunity, since the target antigen is a tumor antigen that occurs physiologically in one's own body, the immune system regards the tumor antigen as its own component and does not generally exhibit an aggressive response. This is because the inherent system of "self-tolerance" that prevents self-reactiveness of T cells causes cell death of immature T cells that recognize self-antigen and strongly react therewith, and functionally suppress the remaining T cells that moderately or weakly react with self-antigen. Therefore, careless administration of a tumor antigen highly possibly induces $T_{reg}$ and suppresses immune responses against tumor. To exclude such possibility, the inventor once successfully induced an antitumor immunity by a method including inducing Th cell against a third party antigen, Bordetella pertussis, by using, as an adjuvant, a non-tumor antigen pertussis vaccine with an HLA class I binding tumor antigen polypeptide that induces CTL, and growing a tumor antigen specific CTL by the Th cell (patent document 1). Particularly, since a Bordetella pertussis whole cell vaccine is used in this method, Th1 type of Th cell showing high CTL induction activity can be efficiently induced. As for the induction of tumor antigen specific Th1 cell, however, a certain induction method does not exist yet due to the problems of regulatory T cells described above.

WT1 tumor-specific antigens which bind an MHC class II molecule, and their use for cancer treatment have been described (patent documents 2 and 3).

[Document List]

[patent document]

patent document 1: WO2008/096831
patent document 2: EP 2423310
patent document 3: WO 2010/123065

[non-patent documents]

non-patent document 1: Fallarino et al., J Immunol 165, 5495-5501, 2000

non-patent document 2: Jin et al., Int J Cancer 111, 558-567, 2004
non-patent document 3: Nishimura et al., J Exp Med 190, 617-627, 1999
non-patent document 4: Xie et al., J Exp Med 207, 651-667, 2010
non-patent document 5: Keene and Forman, J Exp Med 155, 768-782, 1982
non-patent document 6: Bennett et al., Nature 393, 478-480, 1998
non-patent document 7: Ridge et al., Nature 393, 474-478, 1998
non-patent document 8: Kennedy and Celis, J Immunol 177, 2862-2872, 2006
non-patent document 9: Mueller et al., J Immunol 176, 7379-7384, 2006
non-patent document 10: Janssen et al., Nature 852-856, 2003
non-patent document 11: Shedlock and Shen, Science 300, 337-339, 2003
non-patent document 12: Marzo et al., J Immunol Methods 165, 6047-6055, 2000
non-patent document 13: Wong et al., J Immunol 3112-3131, 2008
non-patent document 14: Yamazaki et al., Blood 110, 4293-4302, 2010

## SUMMARY OF THE INVENTION

Problems to be Solved by the Invention

An object of the present invention is to provide a modified molecule of tumor antigen specific Th-inducing polypeptide and an antitumor agent using same.

Means of Solving the Problems

The inventor noted that a serious problem of the conventional immunization protocol including immunization with MHC (Major Histocompatibility Complex) class I binding peptide to induce CTL is that sufficient antitumor activity cannot be achieved even when tumor-specific CTL is present, since it does not sufficiently infiltrate into a tumor tissue. Therefore, the inventor studied the mechanism of infiltration of tumor-specific T cells into a tumor tissue, and noted that regression of solid tumor in vivo is commonly associated with not only CTL in the tumor tissue but also infiltration of Th cell. The previous reports are common on this point. Generally, since T cells (Th and CTL) circulate the whole body on blood, they are prevented by the vascular endothelial cells, and cannot directly contact with the tumor tissue. The inventor found for the first time that vascular endothelial cells uptake apoptotic tumor cell in the vicinity, and present a tumor antigen-derived polypeptide on MHC class II of vascular endothelial cell to invite tumor-specific Th cells into the tumor tissue. The inventor further observed that Th that invaded into the tumor tissue secretes IFN-γ, and IFN-γ induces expression of chemokines (I-TAC, IP-10, Mig) in the vascular endothelial cells, as a result of which infiltration of CTL, that highly expresses CXCR-3 which is the receptor of these IFN-γ dependent chemokines, into the tumor is promoted, whereby the solid tumor is regressed. For this activity, therefore, the tumor-specific Th cell is desirably a Th1 type Th. This finding has made it obvious that the development of a method for efficiently inducing a tumor antigen-specific Th cell is the key technique for developing an immunization protocol that causes regression of a solid tumor. In this application, note was taken of the addition of a modification conferring resistance to an enzyme that degrades an MHC class II binding polypeptide that induces Th digestion to these polypeptides. As a result, the Th induction efficiency could be increased several hundred-folds as mentioned below. It has been clarified that this effect is remarkably advantageous particularly when a living body is immunized in vivo with a tumor antigen polypeptide, and is a modification necessary for inducing Th cell against a tumor antigen which is a self antigen generally difficult to induce Th cell. That is, it was shown that the Th induction efficiency can be strikingly increased by adding the modification to the MHC class II binding polypeptide according to the polypeptide-recognition efficiency of Th cell. Based on these ideas, the inventor conducted many studies, and completed the present invention.

Accordingly, the present invention provides

[1] a modified molecule of an isolated polypeptide, which is derived from a tumor-specific antigen and binds to an MHC class II molecule, wherein the modification is addition of several amino acids to the N terminal side and optionally to the C terminal side of the isolated polypeptide, wherein the amino acid to be added to the N terminal side of the isolated polypeptide is an amino acid sequence represented by the following formula (II):

$$Y1-Y2-Y3 \quad (II)$$

wherein

(1) Y1 shows Pro, Y2 shows several amino acids or a single bond, and Y3 shows one amino acid or a single bond, or
(2) Y1 shows one amino acid except Pro or an amino group, Y2 shows several amino acids or a single bond, and Y3 shows biotinylated Lys,

provided an amino acid sequence wherein the second amino acid from the N terminal is Pro is excluded;
[2] the modified molecule of [1], wherein the amino acid to be added to the N terminal side of the isolated polypeptide is an amino acid sequence represented by the formula (II), wherein

(1) Y1 shows Pro, Y2 shows one amino acid or a single bond, and Y3 shows one amino acid or a single bond, or
(2) Y1 shows one amino acid except Pro or an amino group, Y2 shows one amino acid or a single bond, and Y3 shows biotinylated Lys,

provided an amino acid sequence wherein the second amino acid from the N terminal is Pro is excluded;
[3] the modified molecule of [2], wherein the amino acid to be added to the N terminal side of the isolated polypeptide is an amino acid sequence represented by the formula (II), wherein Y1 shows Pro, Y2 shows Ser, Y3 shows a single bond,
Y1 shows an amino group, Y2 shows Ser, and Y3 shows biotinylated Lys, or
Y1 shows Pro, Y2 shows Ser, and Y3 shows biotinylated Lys;
[4] the modified molecule of [2] or [3], wherein the amino acid to be added to the C terminal side of the isolated polypeptide is an amino acid sequence represented by the following formula (I) :

$$X1-X2-X3-X4 \quad (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows $\beta$-Ala;

[5] a modified molecule of an isolated polypeptide, which is derived from a tumor-specific antigen and binds to an MHC class II molecule, wherein the modification is addition of several amino acids to the C terminal side and optionally to the N terminal side of the isolated polypeptide, wherein the amino acid to be added to the C terminal side of the isolated polypeptide is an amino acid sequence represented by the following formula (I):

$$X1-X2-X3-X4 \quad (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows $\beta$-Ala;

[6] the modified molecule according to [4] or [5], wherein the amino acid to be added to the C terminal side of the isolated polypeptide is an amino acid sequence represented by the formula (I), wherein

(1) X1 shows biotinylated Lys, X2 shows one amino acid or a single bond, X3 shows one amino acid or a single bond, and X4 shows one amino acid or a carboxyl group (provided when X3 is Pro, then X4 shows one amino acid), or

(2) X1 shows a single bond, X2 shows a single bond, X3 shows Pro, and X4 shows one amino acid;

[7] the modified molecule of [6], wherein the amino acid to be added to the C terminal side of the isolated polypeptide is an amino acid sequence represented by the formula (I), wherein X1 shows biotinylated Lys, X2 shows a single bond, X3 shows a single bond, and X4 shows Gly or a carboxyl group,
X1 shows a single bond, X2 shows a single bond, X3 shows Pro, and X4 shows Ala or β-Ala, or X1 shows biotinylated Lys, X2 shows a single bond, X3 shows Pro, and X4 shows β-Ala;

[8] the modified molecule of any one of [1] - [7], wherein the isolated polypeptide comprises an amino acid sequence derived from WT1, PSA, MAGE-3, survivin, CEA, tyrosinase, hepatitis C virus or EB virus, and binds to an MHC class II molecule;

[9] the modified molecule of [8], wherein the isolated polypeptide comprises an amino acid sequence derived from WT1 and binds to an MHC class II molecule;

[10] an isolated polypeptide comprising the amino acid sequence shown by SEQ ID NO: 37, SEQ ID NO: 38 or SEQ ID NO: 40;

[11] an antitumor agent comprising the modified molecule of any one of [1] - [9], or the isolated polypeptide of [10];

[12] the antitumor agent of [11], further comprising an isolated polypeptide which is derived from a tumor-specific antigen and binds to an MHC class I molecule;

[13] the antitumor agent of [11] or [12], further comprising an adjuvant;

[14] the antitumor agent of [13], wherein the adjuvant is a pertussis vaccine;

[15] a composition comprising the modified molecule of any one of [1] - [9] or the isolated polypeptide of [10], an isolated polypeptide which is derived from a tumor-specific antigen and binds to an MHC class I molecule and an adjuvant, for use in the treatment of a tumor;

[16] a method of examining whether a test subject has an MHC class II molecule that binds to an isolated polypeptide contained in a modified molecule, comprising culturing an antigen presenting cell population derived from the test subject and the modified molecule of any one of [1] - [9], and confirming the binding of the antigen presenting cell population and the modified molecule.

Effect of the Invention

Using the modified molecule of a tumor antigen specific Th-inducing polypeptide of the present invention, a tumor-specific antigen can be presented on an MHC class II molecule of an antigen presenting cell remarkably efficiently than before, and a tumor antigen-specific Th cell can be induced more efficiently. As a result, a tumor antigen-specific Th cell can infiltrate into a tumor tissue. Furthermore, provided that this Th cell has infiltrated into a tumor tissue, CTL is lead to infiltrates into the tumor tissue, and kill the tumor cells.

Brief Description of the Drawings

Fig. 1, the upper panel shows the number of PKH-labeled DO11.10 that infiltrated into solid tumors (EL4 tumor and E.G7 (EL4 expressing OVA gene) tumor). The lower panel shows the number of PKH positive cells in the spleen. KO shows MHC class II (I-A$_\beta$$^b$) KO mouse; KO→F1 shows a CBF1 mouse transplanted with the bone marrow of MHC class II KO mouse; and F1→KO shows a bone marrow chimeric mouse which is the reverse thereof.
Fig. 2 shows the fraction of CD69 positive cells among DO11.10 cells after incubation for 24 hr of F2-IA$^d$ cells that had ingested each tumor cell for 2 days, or OVAII-pulsed F2-IA$^d$ cells and DO11.10 cells.
Fig. 3 shows the fraction of CD69 positive cells among DO11.10 cells after incubation for 24 hr of F2-IA$^d$ cells antigen-presented for 3 days with OVA in the presence or absence of chloroquine, and DO11.10 cells.
Fig. 4 shows comparison of the fraction of CD69 positive cells among DO11.10 cells after incubation for 24 hr of F2-IA$^d$ cells that had ingested each tumor cell for 2 days in the presence or absence of chloroquine, or OVAII-pulsed F2-IA$^d$ cells and DO11.10 cells.
Fig. 5 shows the number of DO11.10 cells that migrated through a layer of F2-IA$^d$ cells pulsed with various concentrations of OVAII, and the fraction of the number among DO11.10 cells that recognized F2-IA$^d$ cells treated similarly and became CD69 positive.
Fig. 6 is a microscopic photograph of endothelial cells that have engulfed EL4-EGFP cell or EG7-EGFP cell. Black arrows show endothelial cells containing debris of a tumor cell intracellularly emitting a green EGFP fluorescence. White arrows show endothelial cells that have not incorporated a tumor cell intracellularly emitting a green EGFP fluorescence.

Fig. 7 is a graph showing the number of DO11.10 cells that crawled under the endothelial cell. The total number of counted endothelial cells is shown by n. The vertical bar shows the range of standard deviation (SD). The combinations that showed a statically significant difference of P<0.05 in the measured numbers by Student's t-test are marked with an asterisk.

Fig. 8 shows an antitumor effect in mice immunized with an OVA-derived polypeptide that activates Th or CTL. EL4 is EL4 tumor transplanted to each immunized mouse; EG7 is EG7 tumor transplanted to the same mouse; OVA-I is CTL-inducing peptide; OVA-II is Th-inducing peptide; and λ is the third party antigen peptide.

Fig. 9 shows the number of PKH-labeled OT-1 that infiltrated into the EL4 or E.G7-OVA (EG7) tumor of CBF1 mouse. CXCR3- is CXCR3 gene-deficient CTL; and IFN-γ is IFN-γ gene-deficient Th cell.

Fig. 10 shows an antitumor effect when mice having a tumor mass were adoptively immunized with Th or CTL, or both. The number of mice used for evaluation is 7 for PBS injection group, 7 for DO11.10 single transfer group, 8 for OT-1 single transfer group and 8 for the group transferred with both DO11.10 and OT-1.

Fig. 11 shows the proliferation of DO11.10 cells that recognized a modified molecule of each OVII.

Fig. 12 shows the proliferation of DO11.10 cells, that recognized a modified molecule of each OVII, when a culture medium containing FCS without heat inactivation was used.

Fig. 13 shows the proliferation of DO11.10 cells, that recognized a modified molecule of each OVII, when a culture medium containing heat-inactivated FCS was used.

Fig. 14 shows the proliferation of DO11.10 cells by a modified molecule of each OVII, when a captopril-free medium was used.

Fig. 15 shows the proliferation of DO11.10 cells by a modified molecule of each OVII, when a captopril-containing medium was used.

Fig. 16 shows the proliferation of DO11.10 cells by a modified molecule of each OVII, when a serum-free medium was used.

Fig. 17 shows the bindability of a modified molecule of each biotinylated OVII to an I-A$^d$ molecule expressed on CH1-IA$^d$ cells.

Fig. 18 shows the proliferation of DO11.10 cells by a modified molecule of each biotinylated OVII.

Fig. 19 shows the proliferation h of DO11.10 cells by a modified molecule of each biotinylated OVII.

Fig. 20 shows the proliferation of DO11.10 cells by a modified molecule of each biotinylated or non-biotinylated OVII.

Fig. 21 shows digestion resistance of a modified molecule of each OVII to serum peptidases. In a.-d., mouse serum was used as the serum, in e., human serum was used as the serum and, in f., MCC peptide was used as the core peptide instead of OVII peptide.

Fig. 22 shows a digestion experiment of OVII peptide using a captopril-containing buffer.

Fig. 23 shows a digestion experiment of OVII peptide using a o-phenanthrolin-containing buffer.

Fig. 24 shows a digestion experiment of OVII peptide using a bestatin-containing buffer.

Fig. 25 shows a digestion experiment of a substrate peptide using an inhibitor of the ACE activity of an antigen presenting cell. The horizontal axis shows the incubation time after substrate addition, and the vertical axis shows the fluorescence unit.

Fig. 26 shows the ratio of CD69 positive DO11.10 cells in in vivo immunization using a modified molecule of each OVII.

Fig. 27 shows the ratio of IFN-γ positive Th cells in in vivo immunization using a modified molecule of WA36.

Fig. 28 shows in vitro W332 reactive Th cell induction activity in human peripheral-blood mononuclear cells (PBMCs) having HLA-DR15, by using a modified molecule of W332. Intracellular IFNγ staining and CD4 staining were performed, analyzed by a flow cytometer, and the ratio of intracellular IFNγ positive cells among CD4 positive cells is shown.

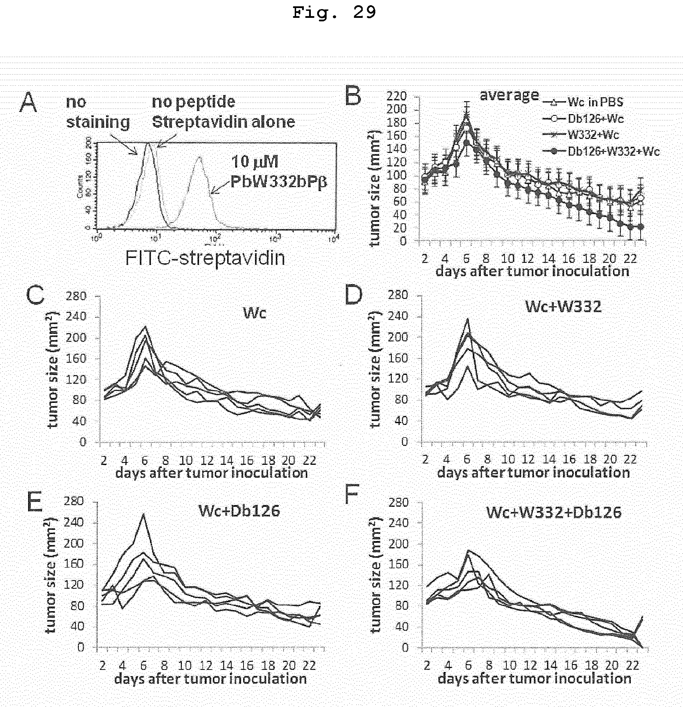

Fig. 29 shows that A: PbW332bPβ binds to CH1-DR4 cell that expresses HLA-DR molecule, and B:C-F each show an average tumor growth curve of 5 mice. The product of the long diameter and the maximum shorter diameter perpendicular thereto of the tumor is shown in the vertical axis. The vertical bar shows the range of standard deviation (SD). C: is a graph showing a tumor growth curve of FBL3 erythroleukemia cells inoculated to CBF1 mice immunized with Bordetella pertussis whole cell vaccine (Wc) alone, which is an adjuvant. D: is a graph showing a growth curve of FBL3 tumor in mice immunized with W332 peptide that induces Wc and WT1 tumor antigen-specific Th cells. E: is a graph showing a growth curve of FBL3 tumor in mice immunized with Db126 peptide that induces CTL recognizing Wc and WT1 tumor antigens. F: is a graph showing a growth curve of FBL3 tumor in mice immunized with both Wc and W332 and Db126.

Fig. 30 shows a competition assay of the binding activity of peptide to MHC class II molecule on a living antigen presenting cell.

Fig. 31 shows a competition assay of the binding activity of peptide to HLA-DR4 molecule on a living antigen presenting cell.

Description of Embodiments

The present inventor has found for the first time that a tumor-specific Th cell recognizes a tumor-specific antigen-derived polypeptide presented on an MHC class II molecule of a vascular endothelial cell and migrates to a tumor tissue. To activate Th cell that recognizes a particular tumor antigen in living organisms, under physiological conditions, a tumor-specific antigen protein needs to be incorporated into an antigen presenting cell (APC) and a polypeptide produced by digestion thereof to be presented to an HLA class II molecule of an antigen presenting cell. It is also possible to bypass the digestion step, and present a polypeptide to an HLA class II molecule APC by directly adding a synthetic peptide. However, when a tumor-specific antigen-derived polypeptide was administered to the targets, the results obtained suggested a possibility that the polypeptide is degraded by a protein (peptide) degrading enzyme present in blood or on the surface of the antigen presenting cell and is not sufficiently presented on the MHC class II molecule. To efficiently present the tumor-specific antigen-derived polypeptide on an antigen presenting cell, the present invention provides a modified molecule (sometimes to be indicated as the modified molecule of the present invention) of an isolated polypeptide (hereinafter sometimes to be indicated as the antigen peptide of the present invention) which is derived from a tumor-specific antigen and binds to an MHC class II molecule.

In the peptide and protein in the present specification, the left end is the N terminal (amino terminal), and the right end is the C terminal (carboxyl terminal) according to the conventional practice in peptide marking.

In the present specification, examples of the tumor include solid malignant tumors such as kidney cancer, lung cancer, esophagus cancer including gastrointestinal cancer, ovarian cancer, prostate cancer, breast cancer, brain tumor, head and neck tumor, sarcoma and the like, leukemia, and blood borne malignant tumor. Of these, a tumor that expresses a tumor-specific antigen can be specifically mentioned. These tumors may be in an advanced stage. The tumor-specific antigen may include not only antigens showing no expression in normal tissue or normal cell but showing expression in tumor tissue or tumor cell, but also an antigen showing a significantly high expression level in tumor tissue or tumor cell as compared to the expression level of normal tissue or normal cell. Examples of such tumor-specific antigen include a protein (peptide) having an activity to induce an immune reaction such as antibody production, cellular immunity and the like. Of these, more preferred is a tumor-specific antigen, from which a polypeptide recognized by an MHC class II molecule is obtained by processing and fragmentation, to induce a T cell (Th1 cell) that stimulates cytotoxic T lymphocytes (CTL). As the isolated polypeptide which is derived from a tumor-specific antigen and binds to an MHC class II molecule, a polypeptide having a length of about 7-20 amino acids, more preferably about 9-17 amino acids, further preferably about 9-15 amino acids, is used. Examples of the tumor-specific antigen processed and recognized by an MHC class II molecule include WT1 (Wilms' tumor-1), PSA (prostate specific antigen), MAGE-3, CEA, survivin, tyrosinase and the like, with preference given to WT1. Alternatively, another tumor-specific antigen recognized by an MHC class II molecule may be a protein derived from a virus that causes the aforementioned tumor when human including mammal is infected therewith. Such antigen refers to a protein derived from a virus group strongly related to the development of a malignant tumor, for example, a protein derived from a virus group strongly related to the development of a malignant tumor such as hepatitis C virus and EB virus or a peptide containing a part of the amino acid sequence thereof. The tumor-specific antigen-derived polypeptide may be a full-length protein or a partial peptide as long as it has the immunogenicity as an antigen. Specifically, as such tumor-specific antigen in case of WT1, those described in WO00/06602, WO00/26249, WO2006/030770 and the like can be preferably used. More specifically, as WT1 antigen-derived polypeptide, W332 (KRYFKLSHLQMHSRKH; SEQ ID NO: 1), WA36 (PVLDFAPPGASAYGS; SEQ ID NO: 2) and the like can be mentioned. As a PSA-derived polypeptide, PSA221-240 (GVLQGITSWGSEPCALPERP; SEQ ID NO: 3) can be preferably used, as a MAGE-3-derived polypeptide, 243-258 (KKLLTQHFVQENYLEY; SEQ ID NO: 4) can be preferably used, as a CEA-derived polypeptide, 321-333 (LWWVNNQSLPVSP; SEQ ID NO: 5) and 653-667 (YACFVSNLATGRNNS; SEQ ID NO: 6) can be preferably used, as a survivin-derived polypeptide, 97-111 (TLGEFLKLDRERAKN; SEQ ID NO: 7) can be preferably used, as a Tyrosinase-derived polypeptide, 175-185 (EIWRDIDFAHE; SEQ ID NO: 8) can be preferably used, more specifically, as a hepatitis C virus peptide, amino acid Nos. 789-797 (ALYGVWPLL; SEQ ID NO: 9), amino acid Nos. 111-130 (DPRRRSRNLGKVIDTFTCGL; SEQ ID NO: 10), amino acid Nos. 161-180 (SVNYATGNLPGCSFSIFLLA; SEQ ID NO: 11) of SEQ ID NO: 184 described in WO 2005/105993 and the like can be preferably used. As an EB virus peptide, LMP-1 159-175 (YLQQNWWTLLVDLLWLL; SEQ ID NO: 12) can be preferably used.

The modified molecule of the present invention is an MHC class II molecule-binding isolated polypeptide modified not to be degraded or not easily degraded by a protein (peptide) degrading enzyme when the antigen peptide of the present invention is administered in vivo or added in vitro. Here, examples of the peptide degrading enzyme include angiotensin I converting enzyme (ACE), dipeptidyl peptidase IV (DPPIV; CD26), aminopeptidase N (CD13), aminopeptidase P and the like. While ACE has an activity to liberate dipeptide from the C terminal of a peptide chain, it cannot liberate dipeptide consisting of Pro-X. DPPIV has an activity to liberate dipeptide (X-Pro) containing a proline residue from a dipeptide having a proline residue at the second position from the N terminal of a peptide chain. While CD13 has an activity to liberate amino acid from the N terminal of a peptide chain, it cannot cleave X-Pro. Amino peptidase P has an activity to liberate amino acid from the N terminal of a peptide chain and also cleaves X-Pro. The modification applied to the antigen peptide of the present invention is not particularly limited as long as the modified molecule is resistant to a protein (peptide) degrading enzyme, presented with an antigen by an MHC class II molecule, and a complex of the MHC class II molecule-the modified molecule of the present invention (or the antigen peptide of the present invention) is recognized by TCR of Th cell. Examples thereof include deletion of several amino acids from the antigen peptide of the present invention, addition of several amino acids to the antigen peptide of the present invention, insertion of several amino acids into the antigen peptide of the present invention, substitution of several amino acids of the antigen peptide of the present invention to other amino acids, and combination thereof, with preference given to addition of several amino acids to the antigen peptide of the present invention. Here, several means 1 or more (1 - about 30, preferably 1 - about 10, more preferably several (1 - 5)) amino acids.

When several amino acids are added to, inserted into or substituted for the antigen peptide of the present invention, the amino acids to be added, inserted or substituted may be 20 kinds of natural amino acids (Gly, Ala, Leu, Ile, Val, Arg, Lys, Glu, Gln, Asp, Asn, Cys, Met, His, Pro, Phe, Tyr, Thr, Ser, Trp) or modified or unnatural amino acids (e.g., biotinylated amino acid (e.g., biotinylated Lys (Lys wherein biotin is covalently bonded to $\varepsilon$ amino group), $N^{\alpha}$-biotinylated amino acid, acetylated amino acid (amino acid wherein N terminal amino group is acetylated), 2-aminoadipic acid, 3-aminoadipic acid, $\beta$-Ala, 2-aminobutyric acid, 4-aminobutyric acid, 6-aminocaproic acid, 2-aminoheptanoic acid, 2,3-diaminopropionic acid, N-ethylglycine, N-ethylasparagine, hydroxylysine, norvaline, norleucine, ornithine etc.). In addition, it may also contain naturally present 20 kinds of $\alpha$-amino acids, $\beta$-amino acids (e.g., $\beta$-Ala), $\gamma$-amino acids and the like. While these amino acids may be hydrophobic or hydrophilic, hydrophilic amino acid is preferable. Here, hydrophobic amino acid means Ile, Leu, Val, Ala, Phe, Pro, Met, Trp, Tyr and Gly, and basic amino acid means Arg, Lys and His, the acidic amino acid means Asp, Glu, and the hydrophilic neutral amino acid means Asn, Gln, Ser, Thr and Cys. Of these amino acids, $\alpha$-amino acid may be an L form or a D form, or a mixture of these, with preference given to an L form. When amino acid is added to an antigen peptide, the position of addition is not particularly limited as long as it is resistant to the degradation by a peptide degrading enzyme and antigen-presented by an MHC class II molecule. It is generally the N terminal and/or C terminal of the antigen peptide of the present invention.

Moreover, several amino acids (peptides) to be added may be straight chain peptide or branched chain (dendrimer type) peptide. For example, when the modified molecule of the present invention contains an amino acid such as Arg, Lys and the like having an amino group in the side chain, a branched chain can be formed by binding the amino group and other amino acid or a carboxyl group of peptide. In addition, when the modified molecule of the present invention contains an amino acid such as Glu, Asp and the like having a carboxyl group in the side chain, a branched chain can be formed by binding the carboxyl group and other amino acid or an amino group of peptide. Furthermore, when the modified molecule of the present invention contains Cys, a branched chain can also be formed via a disulfide bond with other Cys or peptide containing same.

The amino acid that may be added to the C terminal side of the antigen peptide of the present invention is the aforementioned amino acid, preferably an amino acid sequence represented by the following formula (I):

$$X1-X2-X3-X4 \quad (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows $\beta$-Ala).

In the present specification, Xn being a single bond means that an amino acid is substantially absent in Xn, and carboxyl group bonded to an asymmetric carbon atom of the amino acid of X(n-1) and an amino group bonded to an asymmetric carbon atom of the amino acid of X(n+1) are connected by a single bond of a peptide bond. Accordingly, for example, the amino acid sequence of the formula (I) wherein X1 shows biotinylated Lys, X2 shows a single bond, X3 shows Pro and X4 shows $\beta$-Ala is an amino acid sequence represented by biotinylated Lys-Pro-$\beta$-Ala.

More preferably, the amino acid that may be added to the C terminal side of the antigen peptide of the present invention is an amino acid sequence represented by the formula (I), wherein

(1) X1 shows biotinylated Lys, X2 shows one amino acid or a single bond, X3 shows one amino acid or a single bond, and X4 shows one amino acid or a carboxyl group (provided when X3 is Pro, then X4 shows one amino acid), or

(2) X1 shows a single bond, X2 shows a single bond, X3 shows Pro, and X4 shows one amino acid.

Further preferably, the amino acid that may be added to the C terminal side of the antigen peptide of the present invention is an amino acid sequence represented by the formula (I), wherein
X1 shows biotinylated Lys, X2 shows a single bond, X3 shows a single bond, and X4 shows Gly or a carboxyl group,
X1 shows a single bond, X2 shows a single bond, X3 shows Pro, and X4 shows Ala or β-Ala, or
X1 shows biotinylated Lys, X2 shows a single bond, X3 shows Pro, and X4 shows β-Ala, and the like.
Most preferably, the amino acid that may be added to the C terminal side of the antigen peptide of the present invention is an amino acid sequence represented by the formula (I), wherein
X1 shows biotinylated Lys, X2 shows a single bond, X3 shows Pro, and X4 shows β-Ala.
Moreover, the amino acid that may be added to the N terminal side of the antigen peptide of the present invention is the aforementioned amino acid, preferably an amino acid sequence represented by the formula (II):

$$Y1-Y2-Y3 \quad (II)$$

wherein

(1) Y1 shows Pro, Y2 shows several amino acids or a single bond, and Y3 shows one amino acid or a single bond, or
(2) Y1 shows one amino acid except Pro or an amino group, Y2 shows several amino acids or a single bond, and Y3 shows biotinylated Lys

(provided an amino acid sequence wherein the second amino acid from the N terminal is Pro is excluded).
In the present specification, Yn being a single bond means the same as the above-mentioned Xn being a single bond.
More preferably, the amino acid that may be added to the N terminal side of the antigen peptide of the present invention is an amino acid sequence represented by the formula (II): wherein

(1) Y1 shows Pro, Y2 shows one amino acid or a single bond, and Y3 shows one amino acid or a single bond, or
(2) Y1 shows one amino acid except Pro or an amino group, Y2 shows one amino acid or a single bond, and Y3 shows biotinylated Lys

(provided an amino acid sequence wherein the second amino acid from the N terminal is Pro is excluded).
Further preferably, the amino acid that may be added to the N terminal side of the antigen peptide of the present invention is an amino acid sequence represented by the formula (II): wherein
Y1 shows Pro, Y2 shows Ser, Y3 shows a single bond,
Y1 shows an amino group, Y2 shows Ser, and Y3 shows biotinylated Lys, or
Y1 shows Pro, Y2 shows Ser, and Y3 shows biotinylated Lys.
Most preferably, the amino acid that may be added to the N terminal side of the antigen peptide of the present invention is an amino acid sequence represented by the formula (II) wherein Y1 shows Pro, Y2 shows Ser, and Y3 shows biotinylated Lys.
One embodiment of the modified molecule of the present invention is obtained by adding an amino acid sequence as mentioned above to the N terminal and/or the C terminal of an antigen peptide (W332, WA36) derived from the aforementioned WT1 antigen. Specifically, an isolated polypeptide containing an amino acid sequence shown by SEQ ID NO: 37, SEQ ID NO: 38 or SEQ ID NO: 40 can be mentioned as the modified molecule of the present invention.
The C terminal of the modified molecule of the present invention may be any of a carboxyl group (-COOH), carboxylate (-COO-), amide (-CONH$_2$) and ester (-COOR).
As R in the ester, a C$_{1-6}$ alkyl group such as methyl, ethyl, n-propyl, isopropyl, n-butyl and the like; a C$_{3-8}$ cycloalkyl group such as cyclopentyl, cyclohexyl and the like; a C$_{6-12}$ aryl group such as phenyl, α-naphthyl and the like; a phenyl-C$_{1-2}$ alkyl group such as benzyl, phenethyl and the like; a C$_{7-14}$ aralkyl group such as an α-naphthyl-C$_{1-2}$ alkyl group (e.g., α-naphthylmethyl and the like), and the like; a pivaloyloxymethyl group and the like are used.
When the modified molecule of the present invention has a carboxyl group (or carboxylate) at a position besides the C terminal, the carboxyl group may be amidated or esterified in the modified molecule of the present invention. As the ester in this case, the above-mentioned ester at the C terminal and the like are used.
Moreover, the modified molecule of the present invention also includes a molecule wherein an amino group of the N terminal amino acid residue (e.g., methionine residue) is protected by a protecting group (e.g., C$_{1-6}$ acyl group such as C$_{1-6}$ alkanoyl (e.g., formyl group, acetyl group and the like), and the like, or biotinylate and the like), a molecule wherein the N terminal glutamine residue produced by cleavage in vivo is pyroglutamic acid, a molecule wherein a substituent on the side chain of an amino acid in the molecule (for example, -OH, -SH, amino group, imidazole group, indole group, guanidino group and the like) is protected by a suitable protecting group (e.g., $C_{1-6}$ acyl group such as $C_{1-6}$ alkanoyl group (e.g., formyl group, acetyl group and the like) and the like, and the like), and a molecule wherein a sugar chain is bonded or, what is called, composite polypeptide such as glycopeptide and the like and the like.

The modified molecule of the present invention may be a free form or a salt form. As a salt of the modified molecule of the present invention, a physiologically acceptable salt with acid or base can be mentioned, and a physiologically acceptable acid addition salt is particularly preferable. Examples of such salt include salts with inorganic acid (e.g., hydrochloric acid, phosphoric acid, hydrobromic acid, sulfuric acid), salts with organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The modified molecule of the present invention can be produced according to a known peptide synthesis method.

The method of peptide synthesis may be any of, for example, a solid phase synthesis process and a liquid phase synthesis process. The modified molecule can be produced by condensing a partial peptide or amino acid capable of constituting the modified molecule of the present invention with the remaining portion, and removing any protecting group the resultant product may have.

Here, the condensation and the protecting group removal are conducted in accordance with methods known per se, for example, the methods indicated in (i) - (v) below:

i) M. Bodanszky and M.A. Ondetti: Peptide Synthesis, Interscience Publishers, New York (1966)
ii) Schroeder and Luebke: The Peptide, Academic Press, New York (1965)
iii) Nobuo Izumiya, et al.: Peptide Gosei-no-Kiso to Jikken (Basics and experiments of peptide synthesis), published by Maruzen Co. (1975)
iv) Haruaki Yajima and Shunpei Sakakibara: Seikagaku Jikken Koza (Biochemical Experiment) 1, Tanpakushitsu no Kagaku (Chemistry of Proteins) IV, 205 (1977)
v) Haruaki Yajima, ed.: Zoku Iyakuhin no Kaihatsu (A sequel to Development of Pharmaceuticals), Vol. 14, Peptide Synthesis, published by Hirokawa Shoten.

The thus-obtained modified molecule can be purified and isolated by a publicly known method of purification. Here, as examples of the method of purification, solvent extraction, distillation, column chromatography, liquid chromatography, recrystallization, a combination thereof, and the like can be mentioned.

When the modified molecule obtained by the above-described method is a free form, it can be converted to an appropriate salt by a publicly known method or a method based thereon; conversely, when the modified molecule is obtained in the form of a salt, the salt can be converted to a free form or another salt by a publicly known method or a method based thereon.

For the synthesis of the modified molecule of the present invention, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin, PEG-Wang resin and the like can be mentioned. Using such resins, amino acids having $\alpha$-amino groups and side-chain functional groups appropriately protected are condensed on the resin in accordance with the sequence of the desired modified molecule according to various condensation methods known *per se. At* the end of the reaction, the modified molecule of the present invention is excised from the resin and the various protecting groups are removed simultaneously, thus affording the desired modified molecule of the present invention.

For the above-described condensation of protected amino acids, various activation reagents which can be used for protein synthesis can be used, and a carbodiimide is preferably used. As the carbodiimide, DCC, N, N'-diisopropylcarbodiimide, N-ethyl-N'-(3-dimethylaminopropyl)carbodiimide and the like are used. For the activation using these carbodiimides, the protected amino acid, along with a racemation-suppressing additive (for example, HOBt, HOOBt), may be added directly to the resin, or the protected amino acid may be activated in advance as a symmetric acid anhydride or HOBt ester or HOOBt ester and then added to the resin.

Solvents used for the activation of protected amino acids and condensation thereof with a resin can be appropriately selected from among solvents that are known to be usable for protein condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; amines such as pyridine; ethers such as dioxane and tetrahydrofuran; nitriles such as acetonitrile and propionitrile; esters such as methyl acetate and ethyl acetate; suitable mixtures thereof; and the like can be mentioned. Reaction temperature is appropriately selected from the range that is known to be usable for protein binding reactions, and is normally selected from the range of about -20°C to about 50°C. An activated amino acid derivative is normally used from 1.5 to 4 times in excess. When a test using the ninhydrin reaction reveals that the condensation is insufficient, sufficient condensation can be conducted by repeating the condensation reaction without elimination of protecting groups. If the condensation is insufficient even though the reaction is repeated, unreacted amino acids may be acetylated using acetic anhydride or acetylimidazole.

A protecting method and a protecting group for a functional group that should not be involved in the reaction of raw materials, a method of removing the protecting group, a method of activating a functional group involved in the reaction, and the like can be appropriately selected from among publicly known groups or publicly known means.

As examples of the protecting group for an amino group of the raw material, Z, Boc, tertiary pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc, and the like can be used.

A carboxyl group can be protected, for example, by alkyl esterification (for example, linear, branched or cyclic alkyl esterification with methyl, ethyl, propyl, butyl, tertiary butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation, and the like.

The hydroxyl group of serine can be protected by, for example, esterification or etherification. As examples of a group suitable for this esterification, lower alkanoyl groups such as an acetyl group, aroyl groups such as a benzoyl group, and groups derived from carbonic acid such as a benzyloxycarbonyl group and an ethoxycarbonyl group, and the like are used. In addition, as examples of a group suitable for etherification, a benzyl group, a tetrahydropyranyl group, a t-butyl group, and the like can be mentioned.

As examples of the protecting group for the phenolic hydroxyl group of tyrosine, Bzl, Cl$_2$-Bzl, 2-nitrobenzyl, Br-Z, tertiary butyl, and the like can be used.

As examples of the protecting group for the imidazole of histidine, Tos, 4-methoxy-2,3,6-trimethylbenzenesulfonyl, DNP, benzyloxymethyl, Bum, Boc, Trt, Fmoc, and the like are used.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like are used. The elimination reaction by the above-described acid treatment is generally carried out at a temperature of about -20°C to about 40°C; the acid treatment is efficiently conducted by adding a cation scavenger, for example, anisole, phenol, thioanisole, metacresol, paracresol, dimethylsulfide, 1,4-butanedithiol and 1,2-ethanedithiol. Also, a 2,4-dinitrophenyl group used as a protecting group of the imidazole of histidine is removed by thiophenol treatment; a formyl group used as a protecting group of the indole of tryptophan is removed by deprotection by acid treatment in the presence of 1,2-ethanedithiol, 1,4-butanedithiol, or the like, as well as by alkali treatment with a dilute sodium hydroxide solution, dilute ammonia, or the like.

As examples of those obtained by activation of the carboxyl group in the raw material, a corresponding acid anhydride, an azide, an activated ester [an ester with an alcohol (for example, pentachlorophenol, 2,4,5-trichlorophenol, 2,4-dinitrophenol, cyanomethyl alcohol, paranitrophenol, HONB, N-hydroxysuccimide, N-hydroxyphthalimide, or HOBt)] and the like are used. As examples of those obtained by activation of the amino group in the raw material, a corresponding phosphoric amide is used.

In another method of preparing an amide of the modified molecule, for example, the α-carboxyl group of the carboxy terminal amino acid is first amidated and hence protected, and a peptide (protein) chain is elongated to a desired chain length toward the amino group side, thereafter a protein etc. having the protecting group for the N terminal α-amino group of the peptide chain only removed and a protein etc. having the protecting group for the C terminal carboxyl group only removed are prepared, and the both proteins etc. are condensed in a mixed solvent described above. For details about the condensation reaction, the same as above applies. After the protected modified molecule obtained by the condensation is purified, all protecting groups can be removed by the above-described method to yield a desired crude modified molecule. By purifying this crude modified molecule using various publicly known means of purification, and freeze-drying the main fraction, a desired amide of the modified molecule can be prepared.

In order to obtain esters of the modified molecule, a desired ester of the modified molecule can be prepared by, for example, condensing the α-carboxyl group of the carboxy terminal amino acid with a desired alcohol to yield an amino acid ester, and then treating the ester in the same manner as with an amide of the modified molecule.

That the thus-obtained modified molecule has MHC class II molecule bindability can be confirmed by, for example, the methods described in the below-mentioned Examples, or other known method can also be used.

The modified molecule of the present invention is resistant to degradation by protein (peptide) degrading enzymes, and therefore, an antigen presenting cell can efficiently present the modified molecule (or antigen peptide). Hence, the present invention also provides an antitumor agent containing a modified molecule of an isolated polypeptide (the modified molecule of the present invention), which is derived from a tumor-specific antigen, and binds to an MHC class II molecule.

The tumor to be the treatment target of the antitumor agent of the present invention is a tumor that expresses the antigen peptide of the present invention contained in the antitumor agent. Examples of the tumor include solid malignant tumor such as kidney cancer, lung cancer, gastrointestinal cancer including esophagus cancer, ovarian cancer, prostate cancer, breast cancer, brain tumor, head and neck tumor, sarcoma and the like, leukemia, and blood borne malignant tumor.

The animal to be the application subject of the antitumor agent of the present invention is a mammal having an immunity mechanism, namely, human and mammals other than human. Examples of the mammals other than human include monkey, bovine, horse, swine, sheep, rabbit, dog, cat, mouse, rat, hamster, guinea pig and the like. Among these, human is a preferable application subject.

The antitumor agent containing the modified molecule of the present invention is low toxic, and can be administered directly as a liquid or a pharmaceutical composition in a suitable dosage form to human orally, transmucosally (instillation, spray on nasal mucosa, inhalation of aerosol and the like), transdermally, or parenterally (e.g., intravascular administration, subcutaneous administration, intradermal administration and the like).

The pharmaceutical composition used for administration may contain the modified molecule of the present invention, a pharmacologically acceptable carrier, a diluent or an excipient, a surfactant and the like. Such pharmaceutical composition is provided as a dosage form suitable for oral, transmucosal, transdermal or parenteral administration. Moreover, to confer sustained releaseability, it may be adsorbed to or embedded in a substrate.

As a composition for parenteral administration, injection, suppository and the like are used, and the injection may include dosage forms of intravenous injection, subcutaneous injection, intradermal injection, muscular injection, drip injection and the like. Such injections can be prepared according to a known method. A preparation method of injection includes dissolving, suspending or emulsifying the above-mentioned modified molecule of the present invention in an aseptic aqueous solution or oily solution generally used for injection. As an aqueous solution for injection, saline, an isotonic solution containing glucose, other auxiliary agents and the like are used, and they may be used in combination with a suitable solubilizing agents, such as alcohol (e.g., ethanol), polyalcohol (e.g., propylene glycol, polyethylene glycol), nonionic surfactant [e.g., polysorbate80, HCO-50 (polyoxyethylene (50 mol) adduct of hydrogenated castor oil)] and the like. As an oily liquid, sesame oil, soybean oil and the like are used, and benzyl benzoate, benzyl alcohol and the like may be used in combination as a solubilizing agent. A prepared injection is preferably filled in a suitable ampoule. A suppository used for rectal administration may also be prepared by mixing the above-mentioned modified molecule with a general suppository base.

Examples of the composition for oral administration include solid or liquid dosage forms, which are specifically tablet (including sugar-coated tablet, film-coated tablet), pill, granule, powder, capsule (including soft capsule), syrup, emulsion, suspension and the like. Such composition can be prepared according to a known method, and may contain a carrier, a diluent or an excipient generally used in the pharmaceutical field. As the carrier and excipient for tablet, lactose, starch, saccharose, and magnesium stearate are used.

The above-mentioned parenteral or oral pharmaceutical composition is conveniently prepared in a dosage form in a medication unit adapted to the dosage of the active ingredient. Examples of such dosage form in a medication unit include tablet, pill, capsule, injection (ampoule), and suppository. Preferably, the content of the modified molecule is generally 1 - 3 mg of the above-mentioned modified molecule per unit dosage form.

While the dose of an antitumor agent containing the modified molecule of the present invention varies depending on the subject of administration, type of target cancer, symptom, administration route and the like, for example, a single dose of generally about 0.001 mg - 10 g of the modified molecule of the present invention is conveniently administered to an adult about 1 - 5 times, preferably about 1 - 3 times, per day by intravenous injection. In the case of other parenteral administration and oral administration, an amount according thereto can also be administered. When the symptom is particularly grave, it may be increased according to the symptom.

The aforementioned antitumor agent may contain other active ingredient(s) as long as it does not cause an unpreferable interaction when blended with the modified molecule of the present invention. For example, the antitumor agent of the present invention may be used in combination with other medicaments, for example, alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etoposide, irinotecan and the like. The antitumor agent of the present invention and the above-mentioned medicament may be administered to patients simultaneously or in a staggered manner.

In addition, the antitumor agent of the present invention may further contain an isolated polypeptide which is derived from a tumor-specific antigen and binds to an MHC class I molecule. When an MHC class I molecule-binding tumor-specific antigen-derived polypeptide is contained, not only Th cell but also CTL can be induced simultaneously. Here, the MHC class I molecule-binding tumor-specific antigen may be a tumor-specific antigen, from which a polypeptide recognized by an MHC class II molecule is obtained by processing and fragmentation. Examples of such antigen include HER-2/neu, MART-1, NY-ESO-1, Gp-100, MUC-1, p53, prostate-specific antigen (PSA), hTERT, WT1, survivin, CEA, MAGE-3, a protein derived from a group of viruses strongly associated with the onset of malignant tumors and the like. Of these, a specific antigen derived from the same tumor from which the antigen peptide of the present invention is derived is preferable and, for example, WT1 can be mentioned. While the tumor-specific antigen-derived polypeptide may be a full-length protein, it may be a partial peptide as long as it has immunogenicity as an antigen. Specific examples of the WT1 antigen-derived polypeptide include W10 (ALLPAVPSL; SEQ ID NO: 13), W302 (RVPGVAPTL; SEQ ID NO: 14), W292 (GVFRGIQDV; SEQ ID NO: 15) and the like.

While the length of the amino acid sequence of the isolated polypeptide of the present invention, which is derived from a tumor-specific antigen and binds to an MHC class I molecule, is not particularly limited as long as it can be presented by an MHC class I molecule, it is generally about 8 - about 11 amino acids, preferably about 9 amino acids.

Furthermore, the isolated polypeptide, which is derived from a tumor-specific antigen and binds to an MHC class I molecule, may be a modified molecule modified to avoid degradation by a peptide degrading enzyme, like the modified molecule of the present invention. The embodiment of modification may be the same as that for the modified molecule of the present invention mentioned above, and synthesis and separation of the modified molecule can also be performed according to the aforementioned synthesis method and separation method described for the modified molecule of the present invention.

In addition, the antitumor agent of the present invention may further contain an adjuvant. Examples of the adjuvant include pertussis vaccine, Mycobacterium tuberculosis cell wall skeleton (BCG-CWS), non-methylated deoxy CpG, imiquimod, poly-IC, LPS, peptidoglycan, Toll-like receptor ligand, Flt3, $\alpha$Gal-Cer, mineral oil, vegetable oil, alum, aluminum compound, bentonite, silica, muramyl dipeptide derivative, thymosin, interleukin and the like. As the "pertussis vaccine", a whole cell pertussis vaccine comprising a cell wall fraction of formaldehyde-treated or heat-treated Bordetella pertussis, a cell-free pertussis vaccine comprising a component semipurified or purified from Bordetella pertussis (e.g., pertussis toxin, fibrous hemagglutinin and the like) and the like can be mentioned. Regarding how to prepare a pertussis vaccine, a publicly known method can be used without limitations. Commercial products of pertussis vaccines may be used, which are available from, for example, Bio Farma (Indonesia) and the like.

Moreover, the modified molecule of the present invention can be used for testing whether an antigen presenting cell of a test subject expresses an MHC class II molecule to which the antigen peptide of the present invention contained in the modified molecule can be bound. This method enables judgment of whether the test subject can be a subject of administration of the antitumor agent of the present invention containing the modified molecule of the present invention. Accordingly, the present invention also provides a method of examining whether a test subject has an MHC class II molecule that binds to the antigen peptide of the present invention, comprising culturing an antigen presenting cell population derived from the test subject and the modified molecule of the present invention, and confirming the binding of the antigen presenting cell population and the modified molecule.

The test subject who receives the test method of the present invention includes tumor patients to be the subject to be treated with the aforementioned antitumor agent, and those suspected of having such tumor.

The antigen presenting cell population used for the test method of the present invention is not particularly limited as long as it is collected from the above-mentioned test subject. Examples of such antigen presenting cell population include body fluids such as whole blood, lymphatic fluid, brain spinal cord fluid and the like and fractions thereof and the like. Particularly preferred are blood, lymphatic fluid and the like, since they can be recovered rapidly and conveniently, and are less-invasive to the test subject and the like. While the most preferred antigen presenting cell population is a fresh and living cell, it may be lightly fixed with 0.1-1% paraformaldehyde and the like. As a cell population to be used as a control, a cell expressing an MHC class II molecule of a different genotype, and a cell population that does not express an MHC class II molecule can be used.

Whether the modified molecule of the present invention binds to an antigen presenting cell population can be measured by, for example, mixing the both, culturing them for a few hours to overnight, removing free peptide, further letting bind a fluorescence-labeled avidin reagent to biotin peptide bound to an MHC class II molecule on the cell surface, and measuring the amount of fluorescence bound to the cell by flow cytometry.

Using the modification applied to the N terminal and/or the C terminal of the antigen peptide of the present invention, whether any peptide applied with the modification can bind to an antigen presenting cell that expresses a particular MHC class II molecule can be examined. Disclosed is a method of examining whether a peptide can bind to an antigen presenting cell, comprising culturing an antigen presenting cell that expresses a particular MHC class II molecule and any peptide having a modified N terminal and/or C terminal, and confirming the binding of the antigen presenting cell and the peptide.

The antigen presenting cell may be recovered from mammals (preferably human) and the like or an established cell line as long as it expresses a particular MHC class II molecule. While the most preferred antigen presenting cell is a fresh and living cell, it may be lightly fixed with 0.1-1% paraformaldehyde and the like.

As any peptide, a peptide having a length of about 7-20 amino acids, more preferably about 9-17 amino acids, further preferably about 9-15 amino acid, is used. The peptide has a modified N terminal and/or C terminal and the modification to be applied is the above-mentioned modification.

That is, the amino acid that may be added to the C terminal side of the peptide is preferably an amino acid sequence represented by the following formula (I):

$$X1-X2-X3-X4 \quad (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows β-Ala).

More preferably, the amino acid that may be added to the C terminal side of the peptide of the present invention is an amino acid sequence represented by the formula (I), wherein (1) X1 shows biotinylated Lys, X2 shows one amino acid or a single bond, X3 shows one amino acid or a single bond, and X4 shows one amino acid or a carboxyl group (provided when X3 is Pro, then X4 shows one amino acid), or (2) X1 shows a single bond, X2 shows a single bond, X3 shows Pro, and X4 shows one amino acid.

Further preferably, the amino acid that may be added to the C terminal side of the peptide of the present invention is an amino acid sequence represented by the formula (I), wherein X1 shows biotinylated Lys, X2 shows a single bond, X3 shows a single bond, and X4 shows Gly or a carboxyl group,

X1 shows a single bond, X2 shows a single bond, X3 shows Pro, and X4 shows Ala or β-Ala, or

X1 shows biotinylated Lys, X2 shows a single bond, X3 shows Pro, and X4 shows β-Ala, and the like.

Most preferably, the amino acid that may be added to the C terminal side of the peptide of the present invention is an amino acid sequence represented by the formula (I), wherein X1 shows biotinylated Lys, X2 shows a single bond, X3 shows Pro, and X4 shows β-Ala.

Moreover, the amino acid that may be added to the N terminal side of the peptide is the aforementioned amino acid, preferably an amino acid sequence represented by the formula (II) :

$$Y1-Y2-Y3 \quad (II)$$

wherein

(1) Y1 shows Pro, Y2 shows several amino acids or a single bond, and Y3 shows one amino acid or a single bond, or
(2) Y1 shows one amino acid except Pro or an amino group, Y2 shows several amino acids or a single bond, and Y3 shows biotinylated Lys

(provided an amino acid sequence wherein the second amino acid from the N terminal is Pro is excluded).

More preferably, the amino acid that may be added to the N terminal side of the peptide is an amino acid sequence represented by the formula (II): wherein

(1) Y1 shows Pro, Y2 shows one amino acid or a single bond, and Y3 shows one amino acid or a single bond, or
(2) Y1 shows one amino acid except Pro or an amino group, Y2 shows one amino acid or a single bond, and Y3 shows biotinylated Lys

(provided an amino acid sequence wherein the second amino acid from the N terminal is Pro is excluded).

Further preferably, the amino acid that may be added to the N terminal side of the peptide is an amino acid sequence represented by the formula (II): wherein

Y1 shows Pro, Y2 shows Ser, Y3 shows a single bond,

Y1 shows an amino group, Y2 shows Ser, and Y3 shows biotinylated Lys, or

Y1 shows Pro, Y2 shows Ser, and Y3 shows biotinylated Lys.

Most preferably, the amino acid that may be added to the N terminal side of the peptide is an amino acid sequence represented by the formula (II) wherein Y1 shows Pro, Y2 shows Ser, and Y3 shows biotinylated Lys.

A contact method and a culture method of an antigen presenting cell and any modified peptide may follow the methods described in the aforementioned test method. That is, the modification in the present invention is applied to any peptide to confer resistance to digestion enzymes and biotin modification, the peptide is bound as an indicator peptide to an antigen presenting cell, for example, fluorescence-labeled streptavidin is bound thereto, and the binding amount of the indicator peptide can be quantified. Furthermore, a modified peptide having digestion resistance but without biotin modification is added to this binding measurement system to observe competitive inhibition of the binding of the index peptide, whereby the binding activity of a modified peptide of any amino acid sequence to an MHC class II molecule can be quantified for comparison.

In the specification, where bases, amino acids, etc. are denoted by their codes, they are based on conventional codes in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA: | deoxyribonucleic acid |
| cDNA: | complementary deoxyribonucleic acid |
| A: | adenine |
| T: | thymine |
| G: | guanine |
| C: | cytosine |
| RNA: | ribonucleic acid |
| mRNA: | messenger ribonucleic acid |
| dATP: | deoxyadenosine triphosphate |
| dTTP: | deoxythymidine triphosphoric acid |
| dGTP: | deoxyguanosine triphosphoric acid |
| dCTP: | deoxycytidine triphosphate |
| ATP: | adenosine triphosphate |
| EDTA: | ethylenediaminetetraacetic acid |
| SDS: | dodecylsodium sulfate |
| Gly: | glycine |
| Ala: | alanine |
| Val: | valine |
| Leu: | leucine |
| Ile: | isoleucine |
| Ser: | serine |
| Thr: | threonine |
| Cys: | cysteine |
| Met: | methionine |
| Glu: | glutamic acid |
| Asp: | aspartic acid |
| Lys: | lysine |
| Arg: | arginine |
| His: | histidine |
| Phe: | phenylalanine |
| Tyr: | tyrosine |
| Trp: | tryptophan |
| Pro: | proline |
| Asn: | asparagine |
| Gln: | glutamine |
| pGlu: | pyroglutamic acid |
| Sec: | selenocysteine |
| β-Ala: | β-alanine |

Examples

The present invention is explained in more detail in the following by referring to Examples and Reference Examples, which are not to be construed as limitative.

(Cell and mouse)

As tumor cells, EL4 and E.G7-OVA (EG7) which has come to express OVA protein by introducing OVA gene into EL4 were provided from Dr. H. N. Eisen. DO11.10 cells used, which were CD4 T cell clones, were derived from DO11.10 TCR transgenic mouse (DO11.10tg) (Murphy, K. et al., Science 250: 1720-1723, 1990). The DO11.10 cells were stimulated once a week, and cultured with incubated BALB/c-derived spleen cells treated with radiation and 0.75 μM OVAII peptide (ISQAVHAAHAEINEAGR) in 10% FCS DMEM containing 2% rat ConA supernatant (RCS). In addition, AND TCRtg that recognizes MCC peptide bound to I-A$^k$ was assigned by Dr. Nakano. CH1 cells were purchased from ATCC and maintained in 10% FCS-Iscove's MDM (IMDM). CH1-IA$^d$ and CH1-IA$^b$ cells were prepared by retroviral transduction using pMX (assigned by Dr. Kitamura) as an expression vector. These were all inserted in the cDNA of I-A$_\beta$ and I-A$_\alpha$ chains in this order in tandem, with IRES sequence between them. F-2♀(F2) cells as a CBF1 mouse-derived UV transformed cell line were purchased from Riken BioResource Center (Tsukuba). F2-IA$^d$ cells were established in the same manner as with the above-mentioned CH1-IA$^d$ cells.

(Peptide)

OVII (ISQAVHAAHAEINEA; SEQ ID NO: 17) is a peptide obtained by extracting the 323rd-337th amino acid sequence from the already-reported ovalbumin-derived I-A$^d$-binding epitope OVAII 323-339 (ISQAVHAAHAEINEAGR; SEQ ID NO: 16) (Shimonkevitz, R. et al., J Immunol 133: 2067-2074, 1984). MCC (NERADLIAYLKQATK; SEQ ID NO: 42) is the 89th-103rd amino acid sequence of I-A$^k$-binding moth cytochrome C peptide (Buus, S. et al, Science 235: 1353-1358, 1987). The peptide was manually synthesized according to the Fmoc method, and purified by reversed-phase HPLC using C18 column to a purity of not less than 95%. The peptide concentration was determined by MicroBCA assay (Pierce, Rockford, IL) using BSA as the standard. Biotinylated peptide was synthesized using Fmoc-Lys(Biot)-OH. The synthesized peptides are shown in Table 1.

Table 1

| peptide name | amino acid sequence | sequence No. |
|---|---|---|
| OVAII | ISQAVHAAHAEINEAGR | SEQ ID NO: 16 |
| OVII | ISQAVHAAHAEINEA | SEQ ID NO: 17 |
| ac-OVII | acetyl-ISQAVHAAHAEINEA | SEQ ID NO: 18 |
| OVIIβ | ISQAVHAAHAEINEAG$_\beta$A | SEQ ID NO: 19 |
| OVIIPA | ISQAVHAAHAEINEAPA | SEQ ID NO: 20 |
| OVIIPβ | ISQAVHAAHAEINEAP$_\beta$A | SEQ ID NO: 21 |
| POVII | PSISQAVHAAHAEINEA | SEQ ID NO: 22 |
| POVIIPβ | PSISQAVHAAHAEINEAP$_\beta$A | SEQ ID NO: 23 |
| OVII+ | ISQAVHAAHAEINEAR | SEQ ID NO: 24 |
| bOVII+ | SK$^{bio}$ISQAVHAAHAEINEAR | SEQ ID NO: 25 |
| OVII+bG | SKISQAVHAAHAEINEARK$^{bio}$G | SEQ ID NO: 26 |
| bOVII+b | SK$^{bio}$ISQAVHAAHAEINEARK$^{bio}$ | SEQ ID NO: 27 |
| bOVIIbG | SK$^{bio}$ISQAVHAAHAEINEAK$^{bio}$G | SEQ ID NO: 28 |
| bOVII+bG | SK$^{bio}$ISQAVHAAHAEINEARK$^{bio}$G | SEQ ID NO: 29 |
| bOVII+bPβ | SK$^{bio}$ISQAVHAAHAEINEARK$^{bio}$P$_\beta$A | SEQ ID NO: 30 |
| PbOVIIbPβ | PSK$^{bio}$ISQAVHAAHAEINEAK$^{bio}$P$_\beta$A | SEQ ID NO: 31 |
| PbOVII+bPβ | PSK$^{bio}$ISQAVHAAHAEINEARK$^{bio}$P$_\beta$A | SEQ ID NO: 32 |
| KOVII+ | SKISQAVHAAHAEINEAR | SEQ ID NO: 33 |
| KOVII+K | SKISQAVHAAHAEINEARK | SEQ ID NO: 34 |
| MCC | NERADLIAYLKQATK | SEQ ID NO: 35 |
| PbMCCbPβ | PSK$^{bio}$NERADLIAYLKQATKK$^{bio}$P$_\beta$A | SEQ ID NO: 36 |

(continued)

| peptide name | amino acid sequence | sequence No. |
|---|---|---|
| WA36 | PVLDFAPPGASAYGS | SEQ ID NO: 2 |
| PbWA36bPβ | PSK$^{bio}$PVLDFAPPGASAYGSK$^{bio}$P$_β$A | SEQ ID NO: 37 |
| W332 | KRYFKLSHLQMHSRKH | SEQ ID NO: 1 |
| PbW332bPβ | PSK$^{bio}$KRYFKLSHLQMHSRKHK$^{bio}$P$_β$A | SEQ ID NO: 38 |
| PW332Pβ | PSKRYFKLSHLQMHSRKHP$_β$A | SEQ ID NO: 40 |
| bTR174 | PSK$^{bio}$REFKLSKVWRDQK$^{bio}$P$_β$A | SEQ ID NO: 41 |

Each amino acid is indicated with one letter for amino acid. In the sequences, acetyl- is acetyl group, K$^{bio}$ is biotinylated Lys, and pA is β-Ala. The underlined portions show amino acids overlapping with epitope peptides: OVII, MCC, WA36 and W332.

(Antigen presentation of vascular endothelial cell)

EL4 cells were first opsonized with RGD peptide. Next, 5 mg/ml OVA protein was loaded on the cytoplasm by osmotic pressure loading. After sufficient washing, $2\times10^6$ EL4 cells were added to a confluent culture medium of F2-IA$^d$ cells on a 24-well plate (cultured for 2 days in advance in the presence of 500U/ml rmIFN-γ). As a control, EL4 and EG7 mock-loaded with PBS were RGD-modified in the same manner and used. EL4 and other target cells were added to F2-IA$^d$, and the cell mixture was cultured for 24 hr. As for the control well wherein F2-IA$^d$ cells were directly loaded with OVAII peptide, the cells were pulsed with peptide for 2 hr at the end of the culture. After removing the non-adherent cells and debris of dead cells, the surface of the adhered F2-IA$^d$ cells was sufficiently washed. In an antigen presentation inhibitory experiment using chloroquine, 100 μM chloroquine was added 30 min before addition of EL4, EL4 loaded with OVA or 0.25 mg/ml OVA protein. Furthermore, F2-IA$^d$ cells were cultured for 3 days in the continuous presence of chloroquine.

(Detection of antigen presenting marker)

Whether the F2-IA$^d$ cells presented the target antigen polypeptide on the cell surface was evaluated by monitoring the expression of CD69 by DO11.10 cells. The antigen-presenting F2-IA$^d$ cells and DO11.10 cells were mixed and centrifuged to contact the cells with one another. After 6 hr and 24 hr, the cells were stained with biotin-labeled anti CD69 mAb (H1.2F3, BD Biosciences), and the expression of CD69 by DO11.10 cells was detected by flow cytometry (stained positive with FITC-labeled KJ1.26 (anti clonotypic TCR mAb)).

(Cell migration test)

F2-IA$^d$ cells were cultured until confluence on an insert of Transwell plate (BD Labware, Billerica, CA). The cells were cultured in the presence of 500U/ml rmIFN-γ for the last 3 days of culture. In addition, F2-IA$^d$ cells were pulsed with various concentrations of OVAII peptide for the last 2 hr of culture. After the culture, F2-IA$^d$ cells were washed and CD4$^+$ cells were isolated by negative selection (Dynal mouse CD4 negative isolation kit (Invitrogen)) from DO11.10 transgenic spleen cells to not less than 95% purity, which were added by $4\times10^6$ cells per well. The cells were incubated at 37°C, and the number of DO11.10 that migrated under the chamber in 6 hr was counted.

(Peptide-binding assay)

The CH1-IA$^d$ or CH1-IA$^b$ cells were suspended in 2% BSA IMDM to 2x10$^6$ cells/ml. The cell suspension (100 μl) was added to peptide (100 μl) continuously diluted with serum-free IMDM. After incubating at 37°C for 2 hr, 6 hr or 16 hr, the cells were washed and stained with FITC-streptavidin. The amount of peptide bound to the cells was measured by flow cytometry.

(Growth of CD4 T cells)

Each OVII modified peptide having a concentration gradient formed by serially diluting with 2% RCS-containing 10% FCS-DMEM, and 1x10$^5$ cells per well of DO11.10tg or ANDtg-derived spleen cells were placed and incubated in a 96-well U-plate, and the growth of the spleen cells was measured. The cells were incubated at 37°C for 72 hr, $^3$H-thymidine was added, and the cells were recovered 24 hr later for scintillation counting. In the experiment described particularly, FCS was heat inactivated at 56°C for 30 min. In the experiment described particularly, captopril was added to the medium. In some experiments, AIM-V (Invitrogen, Carlsbad, CA) medium was used. In those experiments, $3 \times 10^5$ DO11.10 spleen cells/well were stimulated with each OVII modified peptide.

(Degradation of peptide)

Peptide was digested in TBS containing 10% fresh mouse serum or fresh human serum. After incubation at 37°C for a given time, 1% trifluoro acid was added to remove various serum proteins by precipitation. After short centrifugation, the soluble fraction was analyzed by reversed-phase HPLC using a C18 analysis column. To normalize variation in the total amount of the peptide loaded for each HPLC, the peak of non-protein components contained in the serum, which is absorbed at the wavelength of 220 nm, was used as an internal control. The peak area of undigested peptides was quantitatively monitored by Chromatopac C-R8A (Shimadzu Corp., Kyoto, Japan), and the amount of the remaining peptide was calculated.

(Measurement of ACE activity)

The ACE activity of the cell surface was measured by a modified method of the method described in Sabatini, R. et al., Anal Biochem 363: 255-262, 2007.

(In vivo immunization using Th cell inducing peptide)

CBF1 mouse was immunized with LPS blast of splenocyte pulsed with either λ repressor peptide (LEDARRLKAIYEKKK; SEQ ID NO: 39) or OVAII peptide as a Th epitope peptide, and K$^b$-binding OVA-I peptide, which is a CTL inducing peptide. LPS blast derived from one spleen was pulsed with peptide, and intraperitoneally injected to immunize two mice. The next day of the third immunization, $9 \times 10^6$ EL4 cells and $1.2 \times 10^7$ EG7 cells were intradermally transplanted in a pair on the back skin of the mice. The tumor size was measured every other day. Immunization was performed once again 2 weeks from the tumor transplantation.

(In vivo immunization using modified peptide)

Peptide dissolved in PBS and vaccine of $1 \times 10^7$ heat inactivated Bordetella pertussis whole cells were mixed immediately before immunization and subcutaneously injected to the footpad of BALB/c mouse. The next day (24 hr later), the popliteal and inguinal lymph node were isolated, and CD69$^+$ (H1.2F3, BD Pharmingen) positive DO11.10 (clonotypic anti-TCR mAb KJ1.26$^+$) cells were measured by flow cytometry.

Example 1: Infiltration of tumor-specific Th cell into tumor planted into bone marrow chimeric mouse

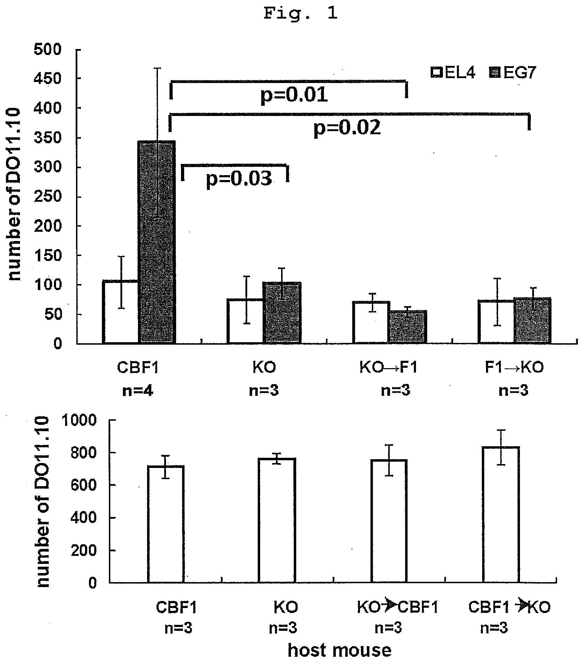

1.5% Oxytetracycline (DENKASEIKEN) was added to the drinking water of CBF1 mouse after 10 Gy gamma-irradiation, I-A$_\beta$$^b$KO mouse after 9.5 Gy gamma-irradiation, or bone marrow chimeric mouse (bone marrow chimeric mouse obtained by transplanting I-A$_\beta$$^b$ KO bone marrow to CBF1 mouse, bone marrow chimeric mouse obtained by transplanting CBF1 mouse bone marrow to I-A$_\beta$$^b$ KO mouse) from one day before $1.5 \times 10^7$ bone marrow transplantation to 14 days after the transplantation. The Th transfer experiment was performed at least 28 days after the bone marrow transplantation. Complete replacement with the donor-derived bone-marrow-derived cells was confirmed by staining peripheral blood mononuclear cells with anti-L$^d$ mAb (30-5-7S), anti-K$^b$ mAb (Y3), anti-I-A$^d$ mAb (39-10-8) and anti-I-A$^b$ mAb (25.9-3). PKH-labeled DO11.10 ($2 \times 10^7$) was transferred on day 6 from the intradermal (left and right) transplantation of tumor cells ($4 \times 10^6$ EL4, and $3 \times 10^6$ EG7) to the back of each one CBF1 mouse, when the tumor diameter grew to about 5-7 mm. Two days later, the tumor was isolated and a 5 μm-thick section was prepared. To prevent duplicate counting of the cells, a specimen was taken every three consecutive sections and the number of PKH-positive cells was counted (Fig. 1). The number of PKH-positive cells observed in 20 fields of a 400-fold fluorescence microscopic image was counted for 10 slices and added.

Example 2: Verification of antigen presentation by vascular endothelial cell

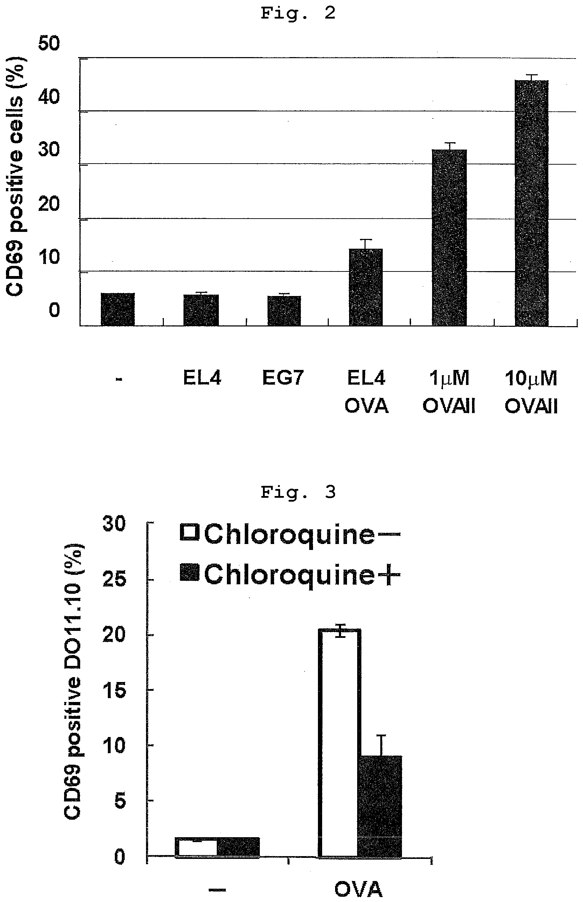

The inventor verified whether vascular endothelial cells (EC) incorporating apoptotic tumor cells can decompose the cytoplasm antigen in the tumor cells, bind polypeptide, which is a degradation product, to an MHC class II molecule, and present the polypeptide. The level of antigen presentation by the F2-IA$^d$ cells was examined by monitoring the expression of CD69, which is an early activation marker on reactive DO11.10 cells. As shown in Fig. 2, the endothelial cells pulsed with OVAII peptide could stimulate DO11.10 cells. When F2-IA$^d$ cells were incubated with opsonized EL4 or EG7, about 30% of the cells incorporated apoptosis tumor cells. However, opsonized EG7 did not induce expression of CD69 in DO11.10 cells even when tried several times. It is considered that this is partly because of the possibility of low concentration of OVA protein in the EG7 cells. F2-IA$^d$ cells pulsed with 0.1 μM OVAII induced expression of CD69 in DO11.10 cells to the same level as when OVA-loaded EL4 was incorporated. While only a considerably small part of the F2-IA$^d$ cells incorporated OVA-loaded EL4, antigen presentation was sufficient for inducing expression of CD69 in DO11.10 cells. As the purity of DO11.10 cells isolated by negative selection from spleen cells and used in this Example, 85% or more was KJ1.26 (mAb specific to clonotypic TCR of DO11.10)-positive, and 95% or more in all CD4 cells was KJ1.26-positive.

Example 3: Influence of inhibition of antigen presentation by extrinsic pathway of endothelial cell on antigen presentation

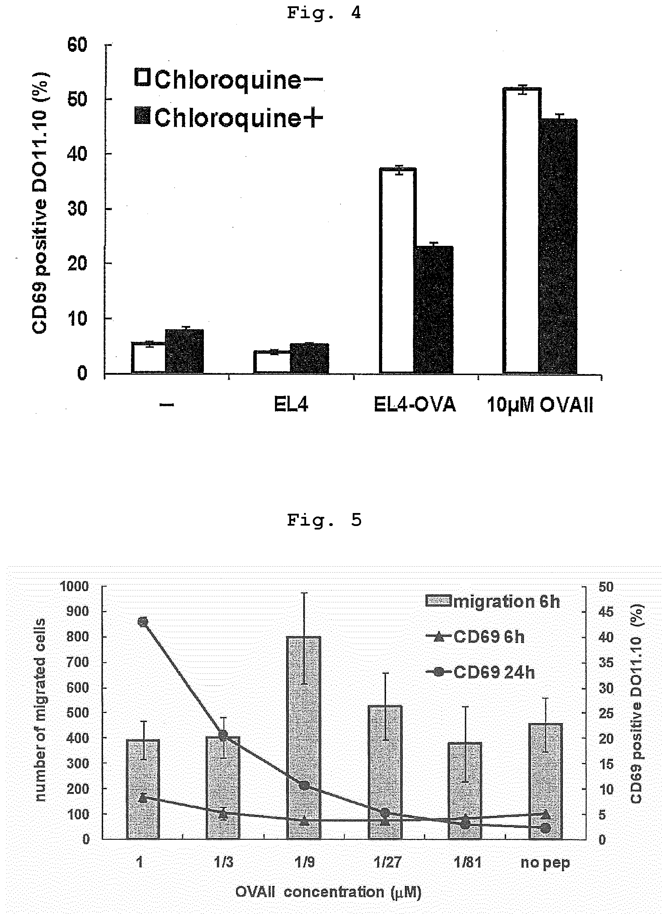

Since a tumor antigen possessed by the incorporated tumor cells is presented by MHC class II molecules, antigen processing via extrinsic pathway was assumed. Therefore, the inventor verified whether antigen presentation is sensitive to chloroquine which is an inhibitor of antigen presentation via endosome. As shown in Figs. 3 and 4, when F2-IA$^d$ cells were treated with chloroquine, presentation of OVA protein known to be also presented in extrinsic pathway decreased. Antigen presentation of the OVA-loaded EL4 was also sensitive to chloroquine. Therefore, antigen presentation of OVA protein present in the EL4 cells by F2-IA$^d$ was suggested to be mainly via the extrinsic pathway.

Example 4: Consideration of whether vascular endothelial cell induces migration of Th cells

The inventor verified whether antigen presentation by endothelial cells could induce migration activity of DO11.10 CD4 T cells to pass through the endothelial cell layer. Before contact with the DO11.10 cells, F2-IA$^d$ cells were plated in advance on the transwell of a plate and pulsed with OVAII peptide. As shown in Fig. 5, when the concentration of the pulsed OVAII peptide was high, DO11.10 cells strongly adhered to F2-IA$^d$ cells, were activated immediately, and did not migrate by penetrating the F2-IA$^d$ cell layer. When the concentration of the pulsed OVAII peptide was low (0.1 μM or less), DO11.10 cells actively migrated, and penetrated the F2-IA$^d$ cell layer. In the low concentration OVAII peptide in this range, induction of the expression of CD69 was hardly confirmed. As examined above, the antigen presentation by the endothelial cells that incorporated EL4-loaded with OVA caused a certain level of CD69-induced expression (Fig. 2), and induction of the expression of CD69, which is comparable thereto, occurred due to the antigen presentation by the endothelial cells pulsed with 0.1 μM OVAII peptide. Therefore, it is presumed that, in the endothelial cells that incorporated OVA-loaded EL4, the antigen was presented at a density comparable to that of the endothelial cells pulsed with 0.1 μM OVAII peptide. Since DO11.10 shows high migration activity on recognition of endothelial cells presenting the antigen at such level of density, the same level of migration activity of DO11.10 is assumed to have been induced also in the endothelial cells that incorporated OVA-loaded EL4.

Example 5: Crawling test of Th cell under endothelial cell isolated from solid tumor

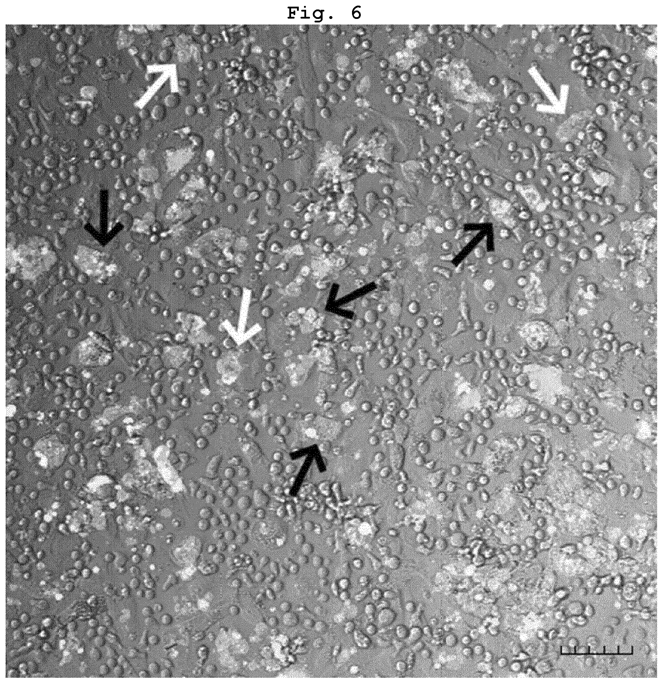

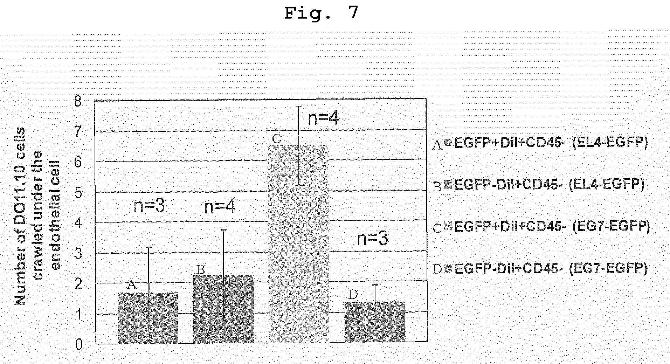

EL4-EGFP or EG7-EGFP cells were inoculated to CBF1 mouse. A grown solid tumor was collected, and digested with 2.5 mg/ml collagenase D (Roche Diagnostics, Indianapolis, IN) dissolved in 20% FCS HBSS (GIBCO, Carlsbad, CA) at 37°C for 3 hr. CD45 positive cells were removed twice with Dynabeads M-280 (Invitrogen, Carlsbad, CA) coated with anti-CD45 mouse antibody 30-F11 (Biolegend, SanDiego, CA). A single cell suspension was cultured on a collagen Type I-coated based dish (AGC Techno Glass Co. Ltd., Chiba, Japan), and the attached cells were used for microscopic observation. Dil-labeled acetylated LDL (Invitrogen) was added at 20 μg/ml, and endocytosis into endothelial cells was performed at 37°C for 4-5 hr. After washing off the acetylated LDL, the cells were stained with biotinylate 30-F11 and Alexa 647-labeled streptavidin (Molecular Probes, Carlsbad, CA). The endothelial cells incorporated Dil-labeled LDL and appeared red. Of the endothelial cells, those that incorporated tumor cell contain debris of the tumor cell emitting green fluorescence of EGFP in the cell, and can be identified (black arrow) as a cell that does not stain in magenta (CD45 positive cell). An endothelial cell that did not incorporate the tumor cell is shown with a white arrow. DO11.10 cells were added, and in vivo movement of DO11.10 cells was observed under a confocal laser microscope FV1000D IX81 (Olympus, Tokyo, Japan), by taking fluorescence images at 37°C every 15 seconds for 1 hr. The microscopic image is shown in Fig. 6. In addition, the number of DO11.10 cells that crawled under the endothelial cells is shown in Fig. 7.

Example 6: Comparison of antitumor activity by in vivo tumor antigen polypeptide immunization

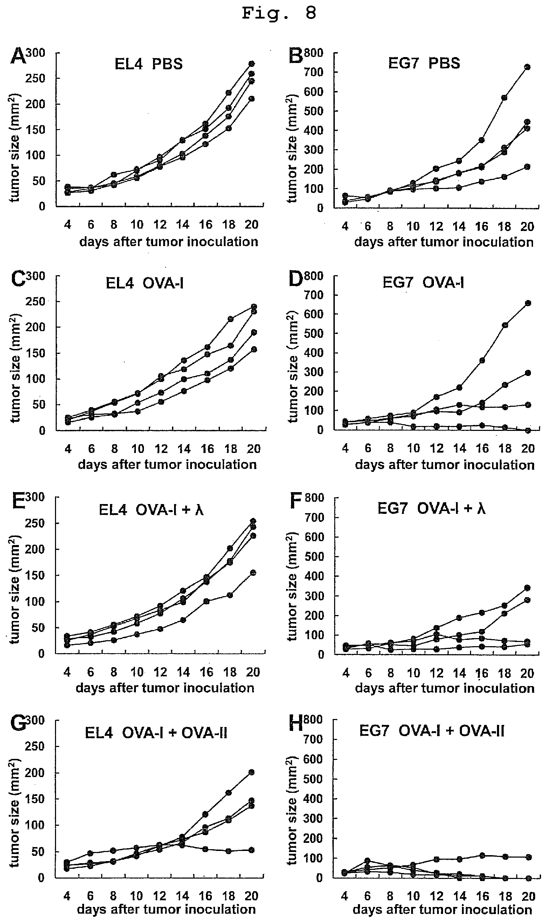

To verify the anti-tumor effect of inducing tumor-specific Th cells in vivo, the inventor compared the immunization protocol that induces 1. CTL alone, 2. CTL and a third party antigen-specific Th cell, and 3. CTL and tumor-specific Th cell. As the antigen presenting cell, LPS blast of spleen cell was used, and the APC loaded with each peptide alone or the mixture of each peptide was injected intraperitoneally 3 times (once per week). On the day of third immunization, $9 \times 10^6$ EL4 and $1.2 \times 10^7$ EG7 tumor were innoculated on the opposite sides of the back, and the tumor diameter was measured from day 4. As shown in Fig. 8, tumor control was only possible to a limited degree by the induction of tumor antigen specific CTL alone by OVA-I peptide. In addition, an immunization protocol inducing, along with CTL, Th cell specific a third party antigen, $\lambda$ peptide by using $\lambda$ peptide, could delay tumor growth as compared to CTL single induction, but the mouse finally died. On the other hand, immunization of mouse with a surrogate tumor antigen, OVAII peptide, together with OVA-I enabled better control of the tumor. The tumor completely disappeared in a part of the mice.

Example 7: Evaluation of promoted infiltration of CTL into tumor

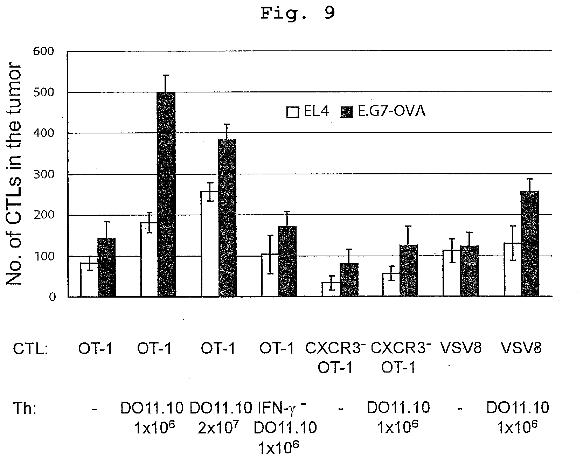

EL4 or EG7 was planted on the right and left respectively, on the back of CBF1 mouse in the same manner as in Example 1, and PKH-labeled OT-1 (CD8 positive cytotoxic T cells (CTL)) cloned from transgenic mouse expressing TCR that recognizes OVA peptide 257-264 bound to $K^b$ ($2 \times 10^7$)) was intravenously injected singly or together with DO11.10 on day 7, and the tumor was isolated 2 days later. A frozen specimen was prepared and the number of PKH positive cells in the 5 $\mu$m-thick section was counted. Nuclear staining with DAPI was added. To prevent duplicate counting, only one section out of three consecutive sections was used as the specimen. In the mouse that received an intravenous injection of DO11.10 ($1 \times 10^6$) showed remarkable infiltration of OT-1 into EG7 (Fig. 9). In the mouse that received DO11.10 ($2 \times 10^7$) non-specific infiltration of OT-1 into the EL4 tumor tended to increase and the infiltration into the EG7 was rather decreased. When IFN-$\gamma$ gene-deficient DO11.10 was transferred, an increase in the DO11.10 dependent infiltration of OT-1 into the tumor was not observed. On the other hand, when CXCR3 gene deficient OT-1 was transferred, the number of infiltration into the tumor decreased, and transfer of DO11.10 scarcely resulted in an increase thereof. When VSV8, which shows different antigen specificity and is a CD8 positive CTL clone that recognizes VSV8 peptide bound to $K^b$, was transferred, the number of infiltration into the EL4 and EG7 tumors did not change, but a promoting effect of DO11.10 on the infiltration of CTL into EG7 was observed, though the level was lower than that of OT-1.

Example 8: Evaluation of antitumor effect of adoptively transferred Th or CTL, or both

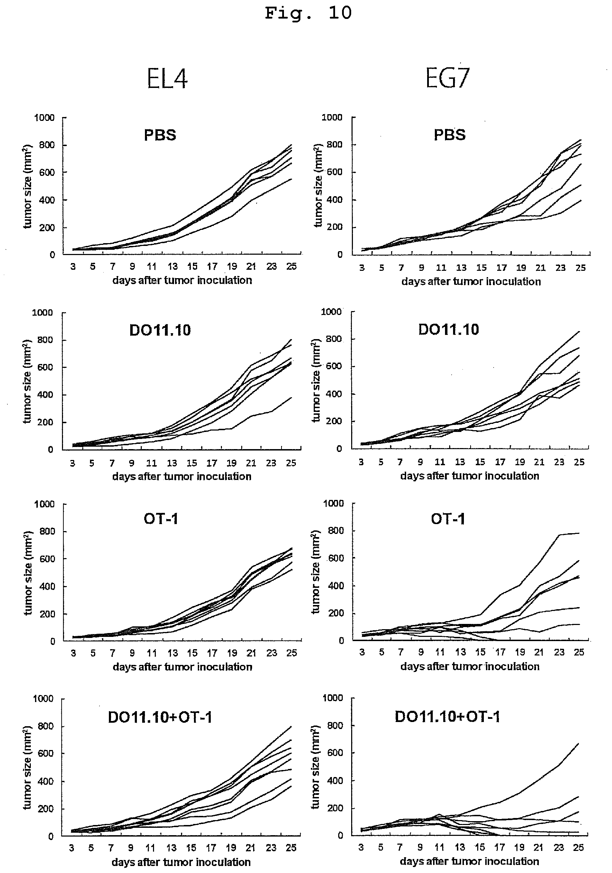

$4 \times 10^6$ EL4 and $3 \times 10^6$ EG7 tumor were planted on the right and left of the back of CBF1 mouse, and adoptive transfer of DO11.10 or OT-1 was started 6 days later when the diameter became 5-7 mm. $1 \times 10^6$ DO11.10 was first injected intravenously, and $2 \times 10^6$ OT-1 was intravenously injected the next day. The tumor diameter was measured every two days. Following the first injection, the adoptive transfer of DO11.10 and OT-1 was each repeated every 5 days (Fig. 10).

Example 9: Design of modified peptide using OVA epitope peptide

The inventor considered from the above-mentioned Examples 1 - 8 that Th infiltrates into the tumor tissue and secretes IFN-$\gamma$, which promotes infiltration of CTL, which highly expresses CXCR-3, a receptor for IFN-$\gamma$ dependent chemokines, into the tumor to cause regression of the solid tumor. Based on such mechanism, the inventor had an idea for a strategy to enhance Th induction efficiency and efficiently activate antitumor immunity, by modifying MHC class II binding polypeptide that induces Th to acquire digestion resistance to degrading enzymes.

The inventor studied modification of the N terminal or C terminal, which provides resistance to protease, on the basis of I-A$^d$ binding ovalbumin (OVA) peptide which is recognized by DO11.10 CD4 T cells. OVII (323-337) is a peptide containing an epitope obtained by removing 2 amino acids from the original OVAII peptide (323-339). In consideration of easy synthesis, OVII was used as a wild-type peptide. Angiotensin I converting enzyme (ACE) has been reported to be a major protease in the serum and involved in the processing of a precursor peptide of MHC class I binding peptide (Sherman, L. et al., J Exp Med 175: 1221-1226, 1992; Kozlowski, S. et al., J Exp Med 175: 1417-1422, 1992). Since ACE is a dipeptidylcarboxy peptidase (Bersanetti, P. et al., Biochemistry 43: 15729-15736, 2004), the inventor designed a peptide having an amino acid at the C-terminal of OVII, and particularly Pro at the second position from the C-terminal thereof to inhibit degradation by ACE enzymes (Table 1). Moreover, since an aminopeptidase having cleavage specificity to the N terminal Pro has not been identified in the enzymes mammals have (Vanhoof, G. et al., FASEB J 9: 736-744, 1995), Pro-Ser dipeptide was added to the N terminal (Table 1). Since Ser is water-soluble and has a smaller side chain, it was assumed to have a slim possibility of physically preventing the antigen recognition by Th and inserted as a spacer. It has been reported that two kinds of dipeptidyl peptidase IV and aminopeptidase P that are expressed on the immunocytes as proline specific aminopeptidases and degrade the N terminal X-Pro as a substrate (Vanhoof, G. et al., FASEB J 9: 736-744, 1995; Yu, D. et al., FEBS J 277: 1126-1144, 2010). The sensitization activity of each modified peptide was measured by means of proliferation curves of DO11.10 cells that recognize an OVA epitope bound to I-A$^d$.

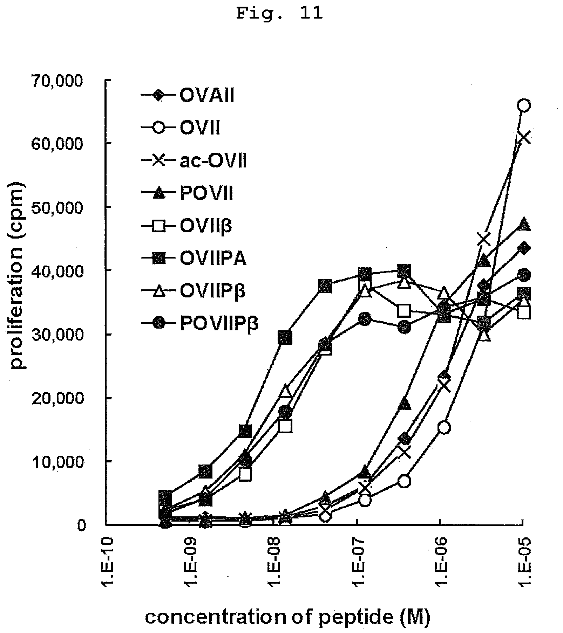

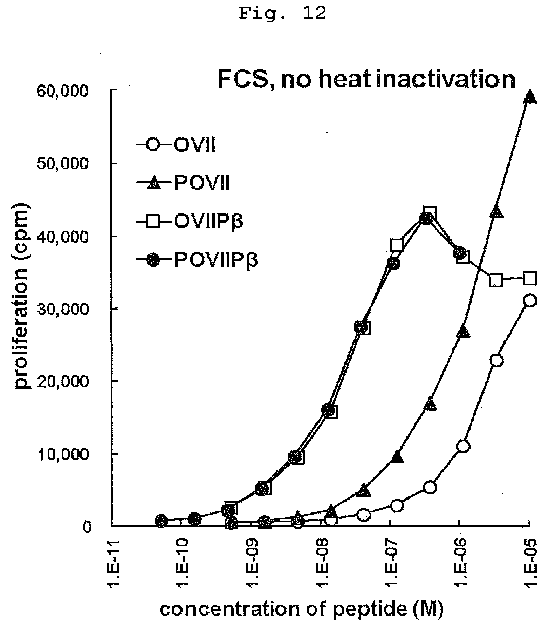

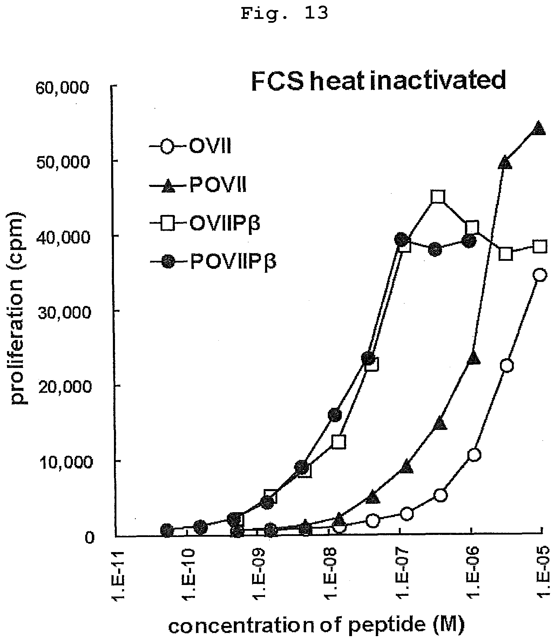

As shown in Fig. 11, a peptide having Pro introduced into the N terminal (POVII) showed about 5-fold increased sensitization activity than OVII. The peptide with modified C-terminal achieved a large increase in the sensitization activity. ACE resistant OVIIPA showed almost 100-fold increase in sensitization activity compared to OVII. A peptide having the C terminal modified with β-Ala was similar to OVIIPA but only showed somewhat lower activity. Acetylation of the N terminal (ac-OVII) showed almost no influence on the sensitization activity. Modification of both the N terminal and C terminal failed to further enhance the sensitization activity. To study whether the effect of these modifications is attributable to the resistance to peptidase in the serum, FCS was heat inactivated. However, the titration curve was not influenced at all (Figs. 12, 13).

Example 10: Investigation of peptide modification using ACE inhibitor

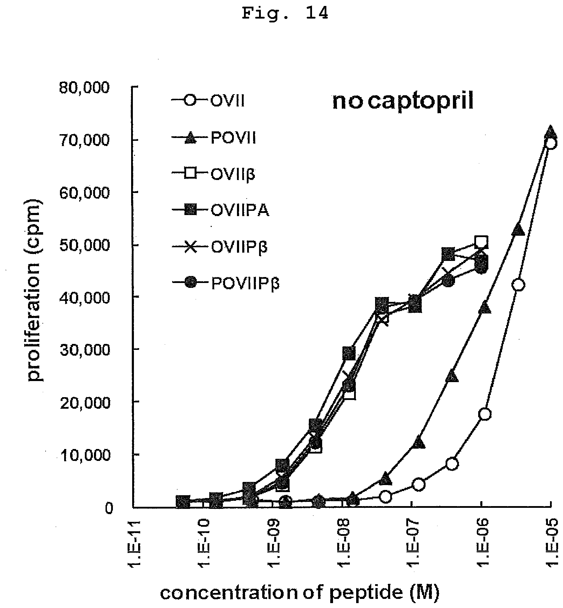

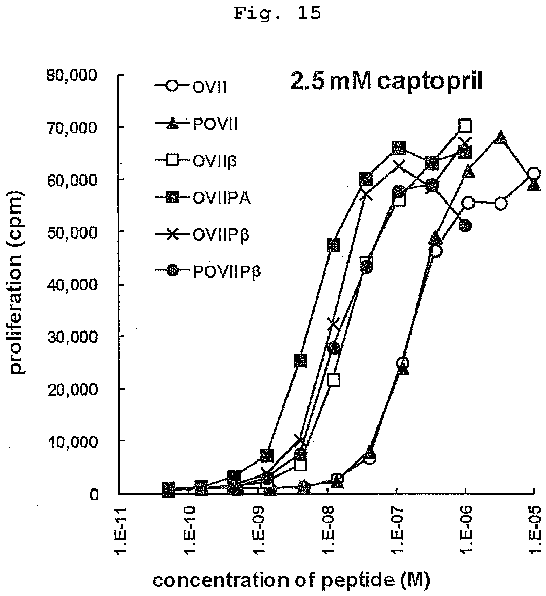

As regards the heat inactivation of enzyme activity, it has been reported that only the C-terminal domain of ACE is sensitive (Voronov, S. et al., FEBS Lett 522: 77-82, 2002), the inventor performed an inhibitory test using captopril having a strong specific inhibitory effect on the both domains of the N terminal domain and C terminal domain of ACE (Dalkas, G. et al., J Pept Sci 16: 91-97, 2009). As shown in Figs. 14, 15, addition of 2.5 mM captopril improved the sensitization activity of OVII to a level close to that of OVIIPA peptide having a protected C terminal. Since DO11.10 cells cannot survive in the presence of 5mM captopril, a test using a higher concentration of captopril was not performed. ACE has been originally reported to be produced as membrane binding extracellular enzyme CD143, and liberated by protease. While endothelial cell strongly expresses ACE, a number of other cell types is also known to express ACE (Coates, D. Int J Biochem Cell Biol 35: 769-773, 2003). Therefore, the inventor predicted that antigen presenting cells (APC) also include a cell that expresses ACE on the cell surface. Of the immunocytes, human dendritic cell has been reported to express ACE (Lapteva, N. et al., Biochem Biophys Res Commun 296: 194-200, 2002); however, there is no report on whether it is involved in the antigen presentation. It has also been reported that monocytes and lymphocytes express ACE only at an ignorable level (Danilov, S. et al., Exp Hematol 31: 1301-1309, 2003).

Example 11: Investigation of peptide modification in serum-free medium

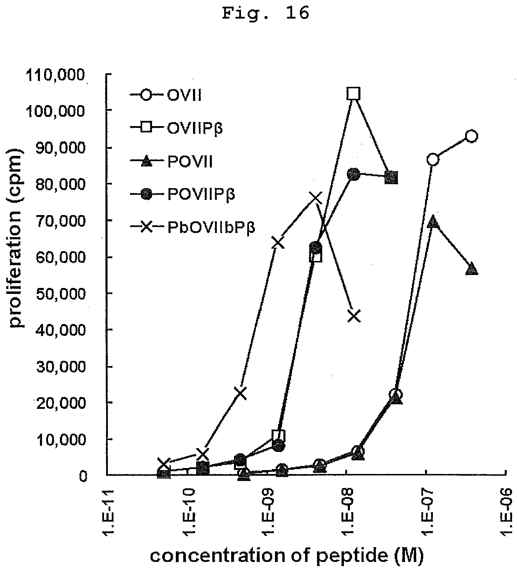

To eliminate the protease activity of a serum, the Th inducing activity of peptide was tested in a serum-free AIM-V medium. Since DO11.10 spleen cells are fragile and tend to attach to the wall of test well in an AIM-V medium, cell-cell contact hardly occurred, due to which proliferation was poor and results were inconsistent. In this Example, therefore, 3-fold more DO11.10 cells were used per well which gave rise to more consistent results. As shown in Fig. 16, removal of the protease in the serum shifted the titration curve of OVII to overlap with that of POVII having modification only of the N terminal. However, other modified molecule peptides still showed high induction activity. This suggests that protease (probably ACE) in the serum exerts a certain level of influence on the presentation of peptide, but a large part of the activity of the modified peptide is associated directly with the antigen presenting cell (APC) or Th cell. Also, biotinylated variant PbOVIIbPβ showed the highest activity. Biotinylated peptide was further studied in the following Examples.

Example 12: High sensitization activity of biotinylated OVII peptide

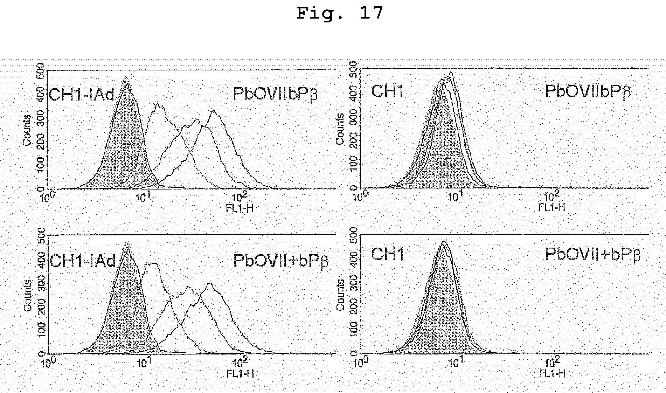

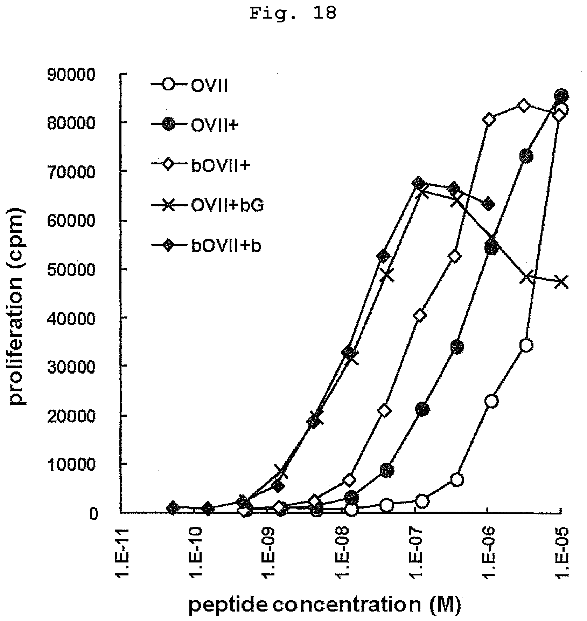

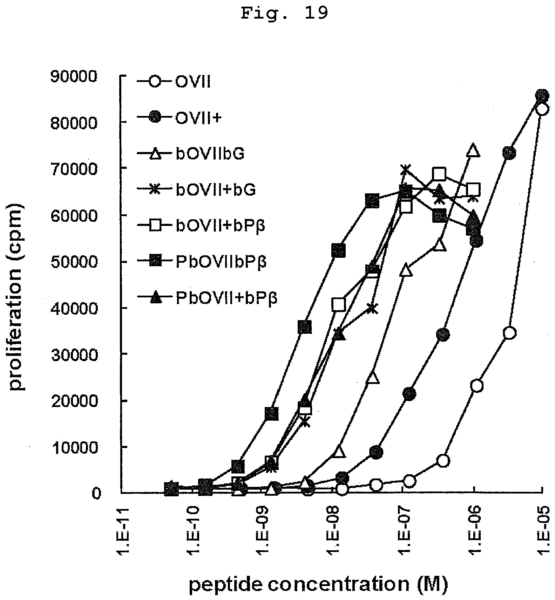

In this Example, the inventor introduced biotinylated Lys (K$^{bio}$) into the inside of the N terminal and/or C terminal of peptide for a labeling purpose. Unexpectedly, the biotinylated peptide showed high sensitization activity, induced a marked proliferation of Th cell. To find the reason therefor, the inventor designed various biotinylated peptides (Table 1), and studied them further. Since biotinylated peptide tends to generally show low solubility, OVII+ wherein Arg is added to the C terminal of OVII was used as the basis of design. The addition of Arg did not affect the binding activity to I-A$^d$ molecule (Fig. 17). As shown in Figs. 18, 19, biotinylated peptide showed very high activity irrespective of whether Pro or β-Ala is present at the terminus, thus clarifying that K$^{bio}$ itself has an effect to enhance sensitization activity. The sensitization activity increased 3-fold merely by introducing K$^{bio}$ into the position of the second amino acid from the N terminal (bOVII+). Introduction of K$^{bio}$ into the C terminal side (bOVII+b) provided a larger effect and increased the sensitization activity 100-fold, as compared to the peptide with modification with Pro at the second position from the C terminal and/or β-Ala at the C terminal (OVIIβ, OVIIPA, Fig. 11). Further addition of Pro-β-Ala to the C terminal provided no effect. On the other hand, addition of Pro to the N terminal further increased the activity 5-fold. The changes in the activity resulting from biotinylation as mentioned above were also seen when DO11.10 cells were cultured under serum-free conditions (Fig. 16). PbOVIIbPβ having modification with K$^{bio}$ on both ends, Pro at the N terminal side and Pro-β-Ala at the C terminal side showed the highest activity. Since K$^{bio}$ confers an additional activity besides the N terminal Pro of PbOVIIbPβ, K$^{bio}$ might have contributed to the increase in the sensitization activity for some reason other than the protease resistance. PbOVIIbPβ also showed 5-fold higher activity than POVIIPβ in an AIM-V serum-free medium (Fig. 16).

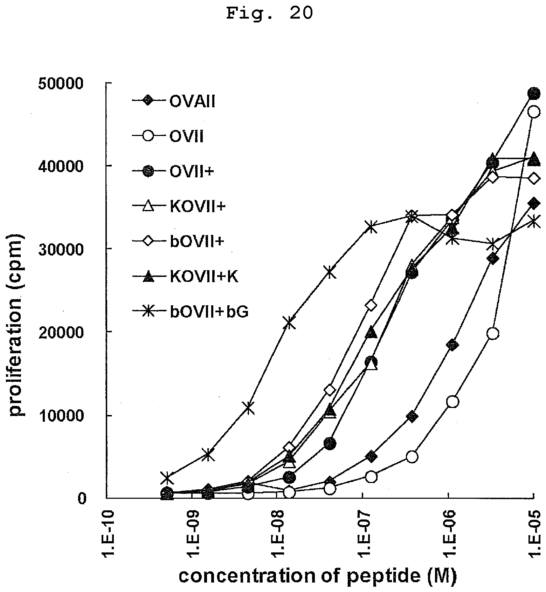

In addition, the inventor also verified whether the sensitization activity of biotinylated OVII peptide was caused by the effect of lysine. As a result, KOVII+ and KOVII+K wherein lysine was added to the N terminal or both terminals of OVII+ showed no difference in the sensitization activity compared to OVII+. On the other hand, bOVII+ wherein biotinylated lysine was added to the N terminal of OVII+, and bOVII+b wherein biotinylated lysine was added to the both terminals showed high activity to induce the proliferation of DO11.10 Th cell, which was about 2-fold for the former and about 10-fold for the latter, relative to KOVII+ and KOVII+K, respectively (Fig. 20).

Example 13: Digestion resistance of peptide to serum peptidase

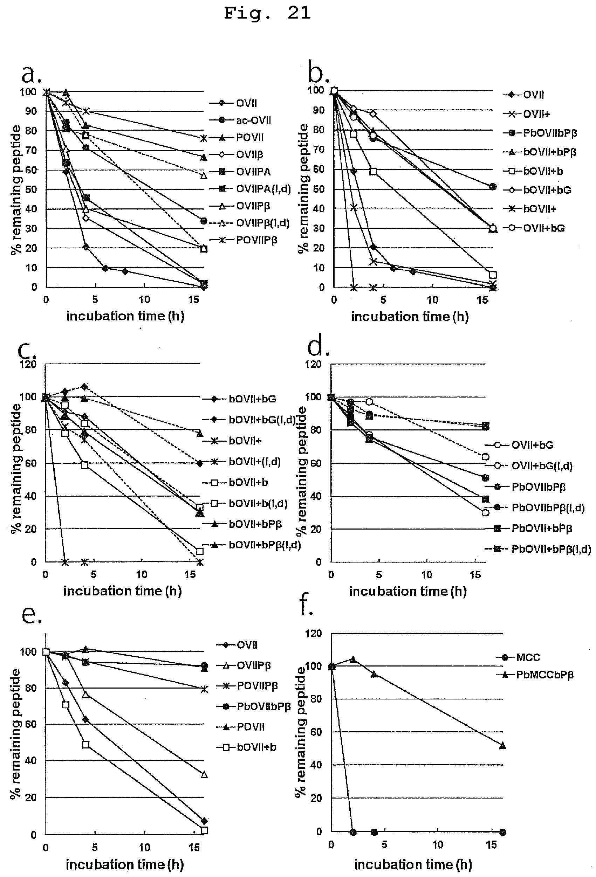

Considering results of the Examples above, it is highly possible that modification confers peptidase resistance to peptide. Therefore, the inventor examined the digestion resistance of peptide in vitro in a 10% fresh mouse serum. The amount of a remaining undigested peptide was quantified by HPLC. As shown in Fig. 21, 40% of OVII was digested within 2 hr, and undigested peptide was not left 6 hr later. It was found that introduction of β-Ala into the C-terminal (OVIIβ) or Pro into the second position from the C terminal (OVIIPA) protects about half of the peptide, which is to be digested within 4 hr, from digestion. When the C-terminal is Pro-β-Ala including both (OVIIPβ), the protection effect was further enhanced, and the amount of the peptide remaining 16 hr later increased. Acetylation of the N terminal (ac-OVII) provided a higher protection effect than the above-mentioned single modification of the C terminal side with Pro-X or β-Ala. When the N terminal is Pro, very strong enzyme resistance was observed (POVII), and almost 70% remained without digestion even after 16 hr. The protective activity against digestion of the N terminal Pro was stronger than Pro-β-Ala as the C terminal, and a peptide having both N, C terminal modifications showed a further additional protective effect (POVIIPβ). Such results appear to be somewhat different from the growth inducing activity of Th shown in Fig. 11. For example, ac-OVII showed higher digestion resistance than OVIIPA and OVIIPβ, but showed no difference from OVII in the Th inducing activity. In the digestion experiment of OVIIPA and OVIIPβ, appearance of the peaks of major degradation products was noted. These peaks were analyzed by mass spectrometry to find that they show peptides lacking Ile from the N terminal and maintaining the epitope. The amount of the residual epitope, which is the sum of these peaks and the peak of undigested peptide, is shown in Fig. 21a with a dotted line. The reason for the high Th growth inducing activity shown by the C terminal-protected peptides can be explained thereby. While POVII showed high digestion resistance in the serum, it showed low activity in the Th proliferation assay. One of the reasons why both ac-OVII and POVII having a protected N terminal showed low Th inducing activity, but the Th inducing activity of the protection at the C terminal was marked may be because DO11.10 is a T cell mainly recognizing the epitope on the C terminal side (Robertson, J. et al., J Immunol 164: 4706-4712, 2000). These results suggest that peptidase activity of the serum affects the antigen presentation efficiency to Th, whereas the peptidase activity accompanying, probably the antigen presenting cell, rather than the serum, greatly influences the antigen presentation of peptide.

The inventor next examined the digestion resistance of biotinylated peptide. After similar incubation in the mouse serum, a modified molecule having $K^{bio}$ added to the C terminal side was found to confer resistance to digestion, as shown in Fig. 21b. bOVII+ having $K^{bio}$ only on the N terminal side appeared to show no digestion resistance. But, a major degradation product remained for a long time, like the aforementioned analysis. When the peak thereof was analyzed by mass spectrometry it was a degradation product wherein 1 or 2 amino acids were removed from the both ends but the core epitope remained intact. A product missing one biotin was commonly seen in all biotinylated peptides. By analysis using an ion trap mass spectrometry, it was found to be mainly a product which has lost the biotin molecule on the N terminal side, at least in the case of PbOVII+bPβ. Besides the above, a product lacking the C terminal Arg in bOVII+, products lacking the N terminal Ser in bOVIIbPβ and OVII+bG, and a product lacking the C terminal $RK^{bio}$ in bOVII+b were mainly observed. When the peaks of these major degradation products are combined with the peak of undigested peptide, the residual amount of epitope was as shown in Fig. 21c, d with a dotted line. The residual amounts of these epitopes correspond well to the Th inducing activity in Figs. 18, 19. In the degradation of peptide, the C terminal $K^{bio}$ generally showed a stronger protection effect on peptidase. Furthermore, to a modified molecule having biotinylated N terminal and biotinylated C terminal, and a modified molecule bearing a combination of the aforementioned N terminal Pro and C terminal Pro-β-Ala an additive resistance against digestion was endowed. However, the reasons remain to be solved why the Th inducing activity of PbOVIIbPβ having $K^{bio}$, was higher by 5-fold than POVIIPb in Fig. 16 as well as the Th inducing activity in the serum free medium while the level of epitope remaining in the serum was almost the same for both peptides. It may be due to the peptidase associated with APC, or by facilitation of antigen presentation by an activity other than digestion resistance.

Similar results were also obtained in human serum (Fig. 21e). A similar protection effect was also observed with the peptide where the core peptide was replaced by MCC (Fig. 21f).

Example 14: Effect of peptidase inhibitor on peptide digestion

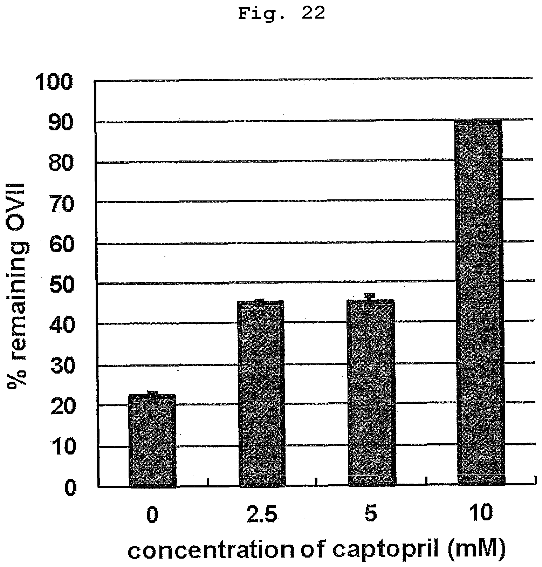

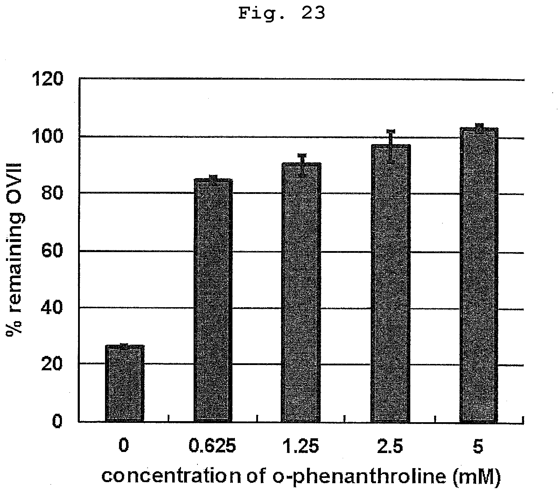

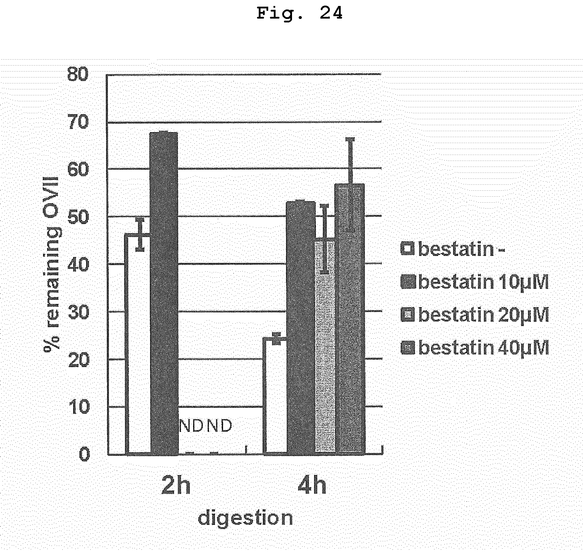

Given the above-mentioned Examples, the inventor examined the effect of peptidase inhibitor on the peptide digestion to identify the peptidase in the serum. Captopril which is a specific inhibitor of ACE was added in the digestion experiment of OVII peptide by using mouse serum, the digestion of peptide was suppressed in a concentration-dependent manner as shown in Fig. 22. As a reason why the inhibitory activity was limited at the lower concentration, it was suspected to be due to a higher sensitivity to ACE of the C-terminal active site of ACE than the N-terminal active site (Dalkas, G. et al., J Pept Sci 16: 91-97, 2009). Furthermore, when o-phenanthrolin which is an inhibitor of metallopeptidase, to which ACE and aminopeptidase in the serum belong, was added, the peptidase activity was almost completely inhibited in a concentration-dependent manner (Fig. 23). Next, bestatin, which is a selective inhibitor of aminopeptidase in the M1 family, was added. As a result, about half of the peptidase activity was suppressed in a concentration-dependent manner (Fig. 24). Therefrom it has been clarified that the peptidase activity in the serum is attributable to aminopeptidase and ACE.

Example 15: Influence of inhibitor on ACE activity of antigen presenting cell

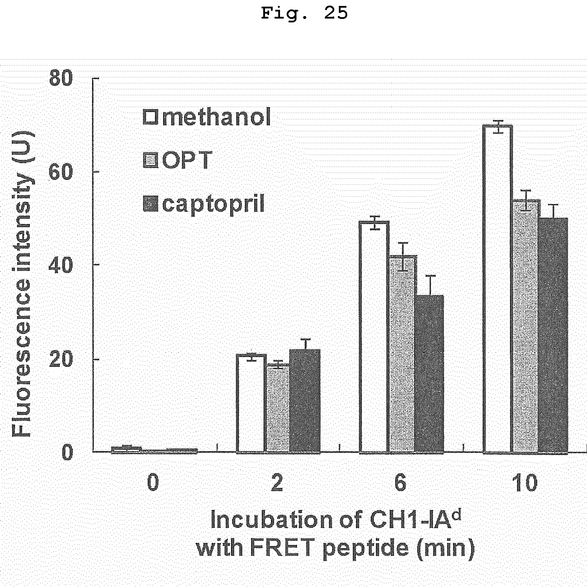

CH1-IA$^d$ cells were suspended in PBS, and incubated in advance in the presence of 2.5 mM o-phenanthrolin or captopril at 37°C for 20 min. 10 $\mu$M Abz-FRK(Dnp)P-OH (Sigma, St. Louis) was added and the cells were incubated at 37°C for a given time. The reaction was quenched by placing them on ice. After centrifugation, fluorescence in the supernatant was measured at excitation wavelength of 320 nm and emission wavelength of 420 nm (Fig. 25). As a result, peptide degrading enzyme activity associated with living cells was confirmed. Furthermore, it was found that the activity thereof is inhibited by o-phenanthrolin, which is an inhibitor of metallopeptidase to which captopril, which is a specific inhibitor of ACE, and ACE belong.

Example 16: Effect of peptide modification for in vivo immunization

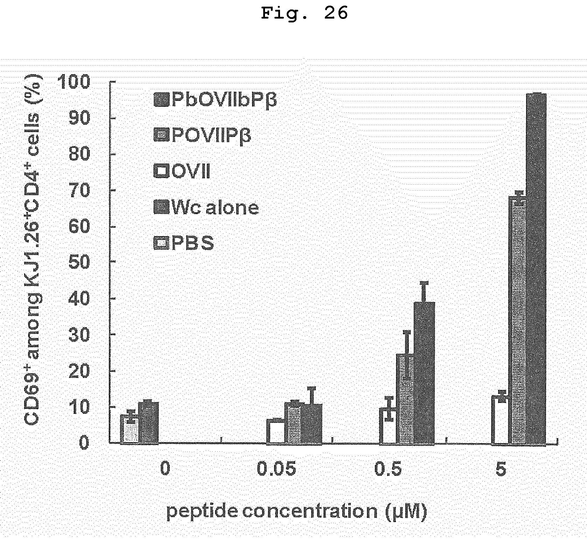

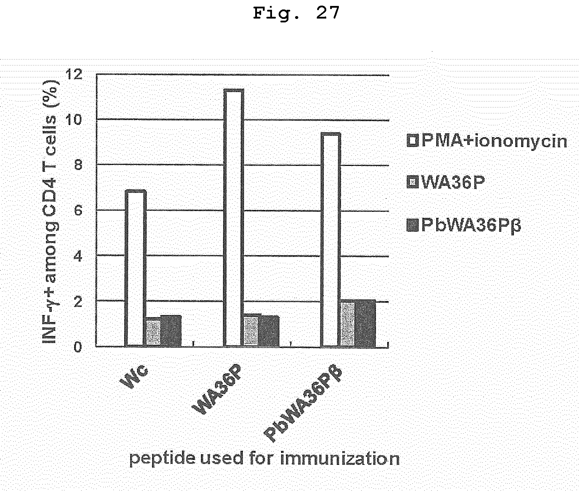

The inventor considered the Th induction activity of modified peptide in in vivo immunization. As shown in Fig. 26, it was found that peptidase resistant POVIIPβ efficiently induces activation of DO11.10 Th cells in in vivo immunization as compared to OVII. PbOVIIbPβ, which is POVIIPβ added with K$^{bio}$, induced activation more efficiently. Furthermore, even when immunized with WT1-derived WA36 peptide, which is a natural tumor antigen, induction of Th cell was extremely difficult with WA36 itself. However, when biotinylated terminal modified peptide was used, Th cell to the tumor antigen could be induced, though weakly (Fig. 27).

Example 17: Tumor antigen peptide induction activity from human peripheral blood mononuclear cell

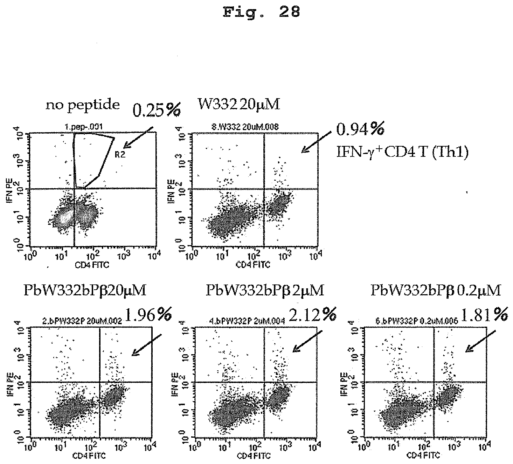

Human-derived peripheral blood mononuclear cells (PBMCs) having HLA-DR15 was stimulated with each concentration of W332 peptide or PbW332bPβ peptide in 10% AB serum-containing DMEM medium. The medium contained 20U/ml recombinant human IL-2 and 0.1 $\mu$g/ml phytohemagglutinin (PHA). One week later, peptide was added to PBMCs of the same subject. The above-mentioned cells were stimulated for the second time with antigen by using an antigen presenting cell bound with the peptide. 4 days later, the above-mentioned respective peptides were added again at each concentration, the cells were stimulated in culture for 4 hr. Brefeldin A was added, and the mixture was further cultured for 1 hr, intracellular IFNγ staining and CD4 staining were performed and the cells were analyzed by flow cytometer. The ratio of intracellular IFNγ positive cells in CD4 positive cells is shown (Fig. 28).

Example 18: in vivo antitumor activity of cancer-bearing mouse immunized with MHC class I peptide and MHC class II peptide

The inventor examined in vivo antitumor activity by inducing Th cell that recognizes Wilms' Tumor 1 (WT1), which is a natural tumor antigen. Induction of Th cell to natural tumor antigen requires careful attention. This is because induction of Th cell to natural tumor antigen may result in the induction of regulatory T cells (Treg) that may conversely attenuate the antitumor activity. In this Example, therefore, Bordetella pertussis whole cell vaccine (Wc) having high immunostimulatory activity was used for immunization to promote induction of Th1 cell.

A peptide derived from WT1 that binds to an MHC class II molecule of mouse has not been identified to date. Thus, the inventor started from searching for such peptide. Human WT1 tumor antigen-derived W332 peptide binds to HLA-DRB1*04:05 molecule and is known to induce human Th cell. Thereafter, W332 was reported to also commonly bind to human HLA-DRB1*15:01, HLA-DRB1*15:02, and HLA-DPB1*09:01. In addition, the amino acid sequence of W332 is identical to the sequence of the corresponding mouse WT1 tumor antigen. It has been clarified that I-A gene, which is one of the mouse MHC class II genes, is an ortholog of human HLA-DR gene. Therefore, being a peptide that commonly binds to multiple human MHC class II molecules, W332 may also bind to mouse I-A molecule, and a binding experiment was performed. PbW332bPβ was added to CH1-IA$^d$ cells and the cells were cultured for 5 hr. The cells were stained with FITC-streptavidin and analyzed by flow cytometry. PbW332bPβ, which is a biotinylated W332 peptide, was found to bind to CH1-DR4 cell that expresses HLA-DR molecule (Fig. 29A).

In the immunization experiment using mouse, the mouse was immunized with PbW332bPβ peptide as a Th-inducing peptide, and Db126 peptide as a CTL inducing peptide. As a tumor to be inoculated to the mouse, FBL3 erythroleukemia cell that highly expresses natural WT1 tumor antigen was used. FBL3 is a cell line that was transformed by infection with Friend leukemia virus, and has high possibility of also expressing virus antigen. In fact, when $2\times10^7$ FBL3 cells were intradermally injected on the back of the mouse, they grew into a solid tumor. However, the tumor had the tendency to shrink naturally after reaching the maximum size. Interestingly, however, the tumor shrank but was not completely rejected, and a small solid tumor remained for at least one month after tumor inoculation. Over time, the tumor sometimes started to grow rapidly in a long-term observation.

CBF1 mouse was immunized in advance by subcutaneous injection of 50 nmoles of Db126 peptide alone, or Db126 peptide and 50 nmoles of W332 peptide, to 4 sites in the top, bottom, left and right of the back. A vaccine of $5\times10^7$ Bordetella pertussis whole cells was added as an adjuvant to all immunization groups including control PBS inoculation group. The mouse was immunized 3 times (once per week) and then $2\times10^7$ FBL3 cells were subcutaneously injected to the back.

Immunization with W332 and Db126 peptide showed stronger antitumor activity than Db126 single peptide immunization. Interestingly, the tumor that remains for a long term in the Wc alone or Db126, or W332 single immunization group tends to shrink in the mouse group immunized with both W332 and Db126 peptide (Fig. 29B-F). Therefrom it is suggested that induction of tumor-specific Th cell together with CTL affords more effective, in vivo antitumor activity.

Example 19: development of a quantitative assay measuring the binding ability to MHC class II molecule

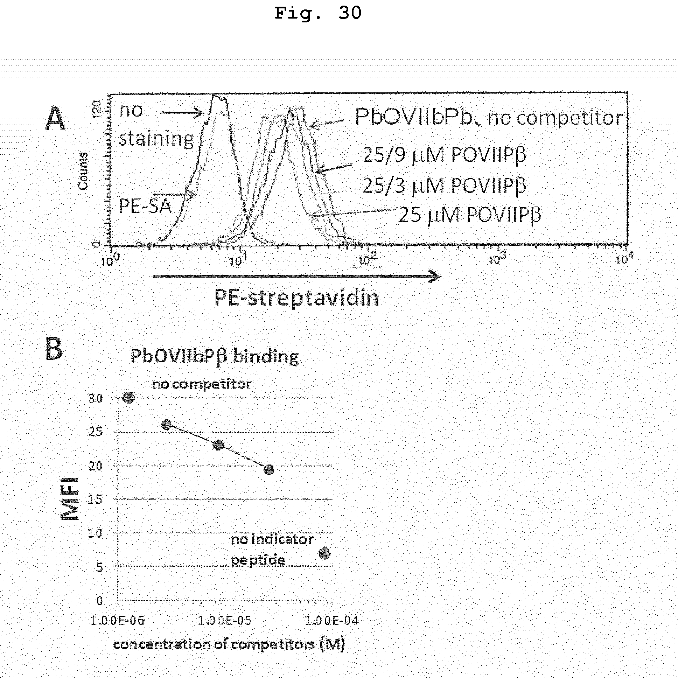

To measure the binding activity of an MHC class II molecule-binding peptide, a method including adding an unlabeled peptide to an MHC class II molecule on a living cell to induce competitive binding of biotinylated indicator peptide to the MHC class II molecule would be available. However, this idea did not permit measurement of binding in a competitive test, since, due to the peptidase activity of living antigen presenting cells, unlabeled peptide is rapidly digested but biotinylation index peptide is difficult to digest. Thus, the inventor examined whether the competitive binding assay becomes possible by adding a modification conferring peptidase resistance to the terminal of the unlabeled peptide to be competed with. 5 μM biotinylated indicator peptide PbOVIIbPβ and serially-diluted POVIIPβ peptide were each added to CH1-IA$^d$ cells, and the mixture was incubated overnight. The cells were stained with PE-streptavidin and measured by a flow cytometer. As a result, a decrease in the binding of biotinylated indicator peptide, which was dependent on the concentration of POVIIPβ peptide added, could be confirmed (Fig. 30A). When the average fluorescence intensity (MFI) of PE-streptavidin was shown graphically, a half maximal inhibition occurred at a concentration of 30 μM (Fig. 30B).

Example 20: quantitative measurement method of peptide binding ability to HLA-DR4 molecule (human MHC class II molecule)

This Example shows that a method of measuring the peptide binding activity to an MHC class II molecule on a living antigen presenting cell can be used not only for the I-Ad molecule, which is a mouse MHC class II molecule, shown in Example 19, but also for a human MHC class II molecule, MHC class II in a similar fashion. In this Example, ρ-chlorophenol (PCP) was added to enable measurement of peptide binding activity by incubating for 4 hr.

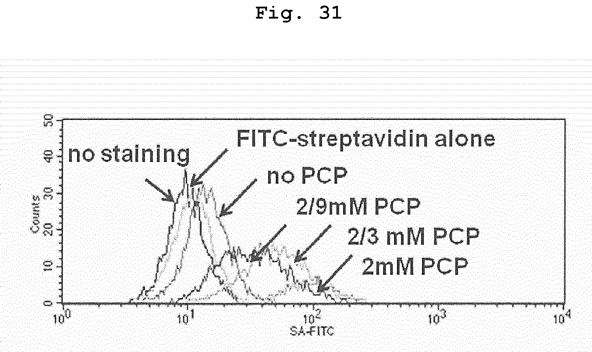

CH1-DRB1*04:05 cells (CH1-DR4 cells) were suspended in 20% FCS IMDM (Iscove's minimum essential medium) at $2\times10^6$/ml, so that 50 μl could provide $1\times10^5$ cells/well in a 96 well U plate. Then, 20 μM bTR174 (PSK$^{bio}$RE-FKLSKVWRDQK$^{bio}$P$_\beta$A: SEQ ID NO: 41) (biotinylation index peptide) was added at 100 μl per well, and IMDM containing 8 mM PCP was further added at 50 μl per well, and finally, CH1-DR4 cells were added at 50 μl ($1\times10^5$/well) and the mixture was incubated at 37°C for 4 hr. Free peptide was removed by centrifugation, the cells were stained with FITC-streptavidin, and measured by flow cytometer. It was found that biotin-labeled bTR174 peptide replaced a peptide endogenously bound to an HLA-DR4 molecule, in a PCP concentration dependent manner, and the amount of the bound biotin-labeled bTR174 peptide can be quantitatively measured (Fig. 31). By adding a competitive peptide, which is generated by adding a peptidase resistant sequence to the terminal of an unlabeled peptide, to this experiment system, the binding activity of the unlabeled peptide can be quantitatively measured.

Industrial Applicability

Using the modified molecule of an MHC class II molecule-binding tumor-specific antigen-derived polypeptide of the present invention, a tumor-specific antigen can be presented on MHC class II molecule of an antigen presenting cell remarkably efficiently than before, and a tumor antigen-specific Th cell can be induced more efficiently.

**Claims**

1. A modified molecule of an isolated polypeptide, which is derived from a tumor-specific antigen and binds to an MHC class II molecule, wherein the modification is addition of several amino acids to the N terminal side and optionally to the C terminal side of the isolated polypeptide, wherein the amino acid to be added to the N terminal side of the isolated polypeptide is an amino acid sequence represented by the following formula (II):

$$Y1\text{-}Y2\text{-}Y3 \ (II)$$

wherein

(1) Y1 shows Pro, Y2 shows several amino acids or a single bond, and Y3 shows one amino acid or a single bond, or
(2) Y1 shows one amino acid except Pro or an amino group, Y2 shows several amino acids or a single bond, and Y3 shows biotinylated Lys,

provided an amino acid sequence wherein the second amino acid from the N terminal is Pro is excluded.

2. The modified molecule according to claim 1, wherein the amino acid to be added to the N terminal side of the isolated polypeptide is an amino acid sequence represented by the formula (II), wherein

(1) Y1 shows Pro, Y2 shows one amino acid or a single bond, and Y3 shows one amino acid or a single bond, or
(2) Y1 shows one amino acid except Pro or an amino group, Y2 shows one amino acid or a single bond, and Y3 shows biotinylated Lys,
provided an amino acid sequence wherein the second amino acid from the N terminal is Pro is excluded.

3. The modified molecule according to claim 2, wherein the amino acid to be added to the N terminal side of the isolated polypeptide is an amino acid sequence represented by the formula (II), wherein
Y1 shows Pro, Y2 shows Ser, Y3 shows a single bond,
Y1 shows an amino group, Y2 shows Ser, and Y3 shows biotinylated Lys, or
Y1 shows Pro, Y2 shows Ser, and Y3 shows biotinylated Lys.

**4.** The modified molecule according to claim 2 or 3, wherein the amino acid to be added to the C terminal side of the isolated polypeptide is an amino acid sequence represented by the following formula (I):

$$X1\text{-}X2\text{-}X3\text{-}X4 \ (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows β-Ala.

**5.** A modified molecule of an isolated polypeptide, which is derived from a tumor-specific antigen and binds to an MHC class II molecule, wherein the modification is addition of several amino acids to the C terminal side and optionally to the N terminal side of the isolated polypeptide, wherein the amino acid to be added to the C terminal side of the isolated polypeptide is an amino acid sequence represented by the following formula (I):

$$X1\text{-}X2\text{-}X3\text{-}X4 \ (I)$$

wherein

(1) X1 shows biotinylated Lys, X2 shows several amino acids or a single bond, X3 shows one amino acid or a single bond, and X4 shows one amino acid or a carboxyl group (provided when X3 is Pro, then X4 shows one amino acid),
(2) X1 shows one amino acid except biotinylated Lys or a single bond, X2 shows several amino acids or a single bond, X3 shows Pro, and X4 shows one amino acid, or
(3) X1 shows one amino acid except biotinylated Lys or a single bond, X2 shows several amino acids or a single bond, X3 shows one amino acid except Pro or a single bond, and X4 shows β-Ala.

**6.** The modified molecule according to claim 4 or 5, wherein the amino acid to be added to the C terminal side of the isolated polypeptide is an amino acid sequence represented by the formula (I), wherein

(1) X1 shows biotinylated Lys, X2 shows one amino acid or a single bond, X3 shows one amino acid or a single bond, and X4 shows one amino acid or a carboxyl group (provided when X3 is Pro, then X4 shows one amino acid), or
(2) X1 shows a single bond, X2 shows a single bond, X3 shows Pro, and X4 shows one amino acid.

**7.** The modified molecule according to claim 6, wherein the amino acid to be added to the C terminal side of the isolated polypeptide is an amino acid sequence represented by the formula (I), wherein
X1 shows biotinylated Lys, X2 shows a single bond, X3 shows a single bond, and X4 shows Gly or a carboxyl group,
X1 shows a single bond, X2 shows a single bond, X3 shows Pro, and X4 shows Ala or β-Ala, or
X1 shows biotinylated Lys, X2 shows a single bond, X3 shows Pro, and X4 shows β-Ala.

**8.** The modified molecule according to any one of the preceding claims, wherein the isolated polypeptide comprises an amino acid sequence derived from WT1, PSA, MAGE-3, survivin, CEA, tyrosinase, hepatitis C virus or EB virus, and binds to an MHC class II molecule.

**9.** The modified molecule according to claim 8, wherein the isolated polypeptide comprises an amino acid sequence derived from WT1 and binds to an MHC class II molecule.

**10.** An isolated polypeptide comprising the amino acid sequence shown by SEQ ID NO: 37, SEQ ID NO: 38 or SEQ ID NO: 40.

11. An antitumor agent comprising the modified molecule according to any one of claims 1 to 9, or the isolated polypeptide according to claim 10.

12. The antitumor agent according to claim 11, further comprising an isolated polypeptide which is derived from a tumor-specific antigen and binds to an MHC class I molecule.

13. The antitumor agent according to claim 11 or 12, further comprising an adjuvant.

14. The antitumor agent according to claim 13, wherein the adjuvant is a pertussis vaccine.

15. A composition comprising the modified molecule according to any one of claims 1 to 9 or the isolated polypeptide according to claim 10, an isolated polypeptide which is derived from a tumor-specific antigen and binds to an MHC class I molecule and an adjuvant, for use in the treatment of a tumor.

16. A method of examining whether a test subject has an MHC class II molecule that binds to an isolated polypeptide contained in a modified molecule, comprising culturing an antigen presenting cell population derived from the test subject and the modified molecule according to any one of claims 1 to 9, and confirming the binding of the antigen presenting cell population and the modified molecule.

**Patentansprüche**

1. Modifiziertes Molekül eines isolierten Polypeptids, das von einem tumorspezifischen Antigen abgeleitet ist und an ein MHC-Klasse-II-Molekül bindet, wobei es sich bei der Modifikation um die Addition mehrerer Aminosäuren an die N-terminale Seite und gegebenenfalls an die C-terminale Seite des isolierten Polypeptids handelt, wobei die an die N-terminale Seite des isolierten Polypeptids zu addierende Aminosäure eine Aminosäuresequenz ist, die durch die folgende Formel (II) dargestellt wird:

$$Y1-Y2-Y3 \qquad (II)$$

wobei

(1) Y1 für Pro steht, Y2 für mehrere Aminosäuren oder eine Einfachbindung steht und Y3 für eine Aminosäure oder eine Einfachbindung steht; oder
(2) Y1 für eine Aminosäure außer Pro oder eine Aminogruppe steht, Y2 für mehrere Aminosäuren oder eine Einfachbindung steht und Y3 für biotinyliertes Lys steht;

mit der Maßgabe, dass eine Aminosäuresequenz, bei der es sich bei der zweiten Aminosäure vom N-Terminus aus um Pro handelt, ausgeschlossen ist.

2. Modifiziertes Molekül gemäß Anspruch 1, wobei die an die N-terminale Seite des isolierten Polypeptids zu addierende Aminosäure eine Aminosäuresequenz ist, die durch die Formel (II) dargestellt wird, wobei

(1) Y1 für Pro steht, Y2 für eine Aminosäure oder eine Einfachbindung steht und Y3 für eine Aminosäure oder eine Einfachbindung steht; oder
(2) Y1 für eine Aminosäure außer Pro oder eine Aminogruppe steht, Y2 für eine Aminosäure oder eine Einfachbindung steht und Y3 für biotinyliertes Lys steht;

mit der Maßgabe, dass eine Aminosäuresequenz, bei der es sich bei der zweiten Aminosäure vom N-Terminus aus um Pro handelt, ausgeschlossen ist.

3. Modifiziertes Molekül gemäß Anspruch 2, wobei die an die N-terminale Seite des isolierten Polypeptids zu addierende Aminosäure eine Aminosäuresequenz ist, die durch die Formel (II) dargestellt wird, wobei
Y1 für Pro steht, Y2 für Ser steht, Y3 für eine Einfachbindung steht;
Y1 für eine Aminogruppe steht, Y2 für Ser steht und Y3 für biotinyliertes Lys steht; oder
Y1 für Pro steht, Y2 für Ser steht und Y3 für biotinyliertes Lys steht.

4. Modifiziertes Molekül gemäß Anspruch 2 oder 3, wobei die an die C-terminale Seite des isolierten Polypeptids zu addierende Aminosäure eine Aminosäuresequenz ist, die durch die folgende Formel (I) dargestellt wird:

$$X1-X2-X3-X4 \qquad (I),$$

wobei

(1) X1 für biotinyliertes Lys steht, X2 für mehrere Aminosäuren oder eine Einfachbindung steht, X3 für eine Aminosäure oder eine Einfachbindung steht und X4 für eine Aminosäure oder eine Carboxygruppe steht (mit der Maßgabe, dass dann, wenn X3 = Pro ist, X4 für eine Aminosäure steht);
(2) X1 für eine Aminosäure außer biotinyliertes Lys oder eine Einfachbindung steht, X2 für mehrere Aminosäuren oder eine Einfachbindung steht, X3 für Pro steht und X4 für eine Aminosäure steht; oder
(3) X1 für eine Aminosäure außer biotinyliertes Lys oder eine Einfachbindung steht, X2 für mehrere Aminosäuren oder eine Einfachbindung steht, X3 für eine Aminosäure außer Pro oder eine Einfachbindung steht und X4 für β-Ala steht.

5. Modifiziertes Molekül eines isolierten Polypeptids, das von einem tumorspezifischen Antigen abgeleitet ist und an ein MHC-Klasse-II-Molekül bindet, wobei es sich bei der Modifikation um die Addition mehrerer Aminosäuren an die C-terminale Seite und gegebenenfalls an die N-terminale Seite des isolierten Polypeptids handelt, wobei die an die C-terminale Seite des isolierten Polypeptids zu addierende Aminosäure eine Aminosäuresequenz ist, die durch die folgende Formel (I) dargestellt wird:

$$X1-X2-X3-X4 \qquad (I),$$

wobei

(1) X1 für biotinyliertes Lys steht, X2 für mehrere Aminosäuren oder eine Einfachbindung steht, X3 für eine Aminosäure oder eine Einfachbindung steht und X4 für eine Aminosäure oder eine Carboxygruppe steht (mit der Maßgabe, dass dann, wenn X3 = Pro ist, X4 für eine Aminosäure steht);
(2) X1 für eine Aminosäure außer biotinyliertes Lys oder eine Einfachbindung steht, X2 für mehrere Aminosäuren oder eine Einfachbindung steht, X3 für Pro steht und X4 für eine Aminosäure steht; oder
(3) X1 für eine Aminosäure außer biotinyliertes Lys oder eine Einfachbindung steht, X2 für mehrere Aminosäuren oder eine Einfachbindung steht, X3 für eine Aminosäure außer Pro oder eine Einfachbindung steht und X4 für β-Ala steht.

6. Modifiziertes Molekül gemäß Anspruch 4 oder 5, wobei die an die C-terminale Seite des isolierten Polypeptids zu addierende Aminosäure eine Aminosäuresequenz ist, die durch die Formel (I) dargestellt wird, wobei

(1) X1 für biotinyliertes Lys steht, X2 für eine Aminosäure oder eine Einfachbindung steht, X3 für eine Aminosäure oder eine Einfachbindung steht und X4 für eine Aminosäure oder eine Carboxygruppe steht (mit der Maßgabe, dass dann, wenn X3 = Pro ist, X4 für eine Aminosäure steht); oder
(2) X1 für eine Einfachbindung steht, X2 für eine Einfachbindung steht, X3 für Pro steht und X4 für eine Aminosäure steht.

7. Modifiziertes Molekül gemäß Anspruch 6, wobei die an die C-terminale Seite des isolierten Polypeptids zu addierende Aminosäure eine Aminosäuresequenz ist, die durch die Formel (I) dargestellt wird, wobei
X1 für biotinyliertes Lys steht, X2 für eine Einfachbindung steht, X3 für eine Einfachbindung steht und X4 für Gly oder eine Carboxygruppe steht;
X1 für eine Einfachbindung steht, X2 für eine Einfachbindung steht, X3 für Pro steht und X4 für Ala oder β-Ala steht; oder
X1 für biotinyliertes Lys steht, X2 für eine Einfachbindung steht, X3 für Pro steht und X4 für β-Ala steht.

8. Modifiziertes Molekül gemäß einem der vorstehenden Ansprüche, wobei das isolierte Polypeptid eine Aminosäuresequenz umfasst, die von WT1, PSA, MAGE-3, Survivin, CEA, Tyrosinase, Hepatitis-C-Virus oder EB-Virus abgeleitet ist und an ein MHC-Klasse-II-Molekül bindet.

# EP 2 792 745 B1

9. Modifiziertes Molekül gemäß Anspruch 8, wobei das isolierte Polypeptid eine Aminosäuresequenz umfasst, die von WT1 abgeleitet ist und an ein MHC-Klasse-II-Molekül bindet.

10. Isoliertes Polypeptid, das die Aminosäuresequenz umfasst, die in SEQ ID Nr. 37, SEQ ID Nr. 38 oder SEQ ID Nr. 40 gezeigt ist.

11. Antitumormittel, das das modifizierte Molekül gemäß einem der Ansprüche 1 bis 9 oder das isolierte Polypeptid gemäß Anspruch 10 umfasst.

12. Antitumormittel gemäß Anspruch 11, das weiterhin ein isoliertes Polypeptid umfasst, das von einem tumorspezifischen Antigen abgeleitet ist und an ein MHC-Klasse-I-Molekül bindet.

13. Antitumormittel gemäß Anspruch 11 oder 12, das weiterhin ein Adjuvans umfasst.

14. Antitumormittel gemäß Anspruch 13, wobei das Adjuvans ein Pertussis-Impfstoff ist.

15. Zusammensetzung, die das modifizierte Molekül gemäß einem der Ansprüche 1 bis 9 oder das isolierte Polypeptid gemäß Anspruch 10, ein isoliertes Polypeptid, das von einem tumorspezifischen Antigen abgeleitet ist und an ein MHC-Klasse-I-Molekül bindet, und ein Adjuvans umfasst, zur Verwendung bei der Behandlung eines Tumors.

16. Verfahren zum Untersuchen, ob ein Proband ein MHC-Klasse-II-Molekül aufweist, das an ein in einem modifizierten Molekül enthaltenes isoliertes Polypeptid bindet, umfassend das Kultivieren einer antigenpräsentierenden Zellpopulation, die von dem Probanden abgeleitet ist, und des modifizierten Moleküls gemäß einem der Ansprüche 1 bis 9 und das Bestätigen der Bindung der antigenpräsentierenden Zellpopulation und des modifizierten Moleküls.

## Revendications

1. Molécule modifiée d'un polypeptide isolé, qui est dérivée d'un antigène spécifique d'une tumeur et se lie à une molécule du CMH de classe II, dans laquelle la modification est l'addition de plusieurs acides aminés au côté N-terminal et éventuellement au côté C-terminal du polypeptide isolé, dans laquelle l'acide aminé à ajouter au côté N-terminal du polypeptide isolé est une séquence d'acides aminés représentée par la formule (II) suivante :

$$Y1-Y2-Y3 \ (II)$$

dans laquelle

(1) Y1 représente Pro, Y2 représente plusieurs acides aminés ou une liaison simple, et Y3 représente un acide aminé ou une liaison simple, ou
(2) Y1 représente un acide aminé à l'exception de Pro ou un groupe amino, Y2 représente plusieurs acides aminés ou une liaison simple, et Y3 représente Lys biotinylée,

à condition qu'une séquence d'acides aminés dans laquelle le deuxième acide aminé à partir de l'extrémité N-terminale est Pro soit exclue.

2. Molécule modifiée selon la revendication 1, dans laquelle l'acide aminé à ajouter au côté N-terminal du polypeptide isolé est une séquence d'acides aminés représentée par la formule (II), dans laquelle

(1) Y1 représente Pro, Y2 représente un acide aminé ou une liaison simple, et Y3 représente un acide aminé ou une liaison simple, ou
(2) Y1 représente un acide aminé à l'exception de Pro ou un groupe amino, Y2 représente un acide aminé ou une liaison simple, et Y3 représente Lys biotinylée,

à condition qu'une séquence d'acides aminés dans laquelle le deuxième acide aminé à partir de l'extrémité N-terminale est Pro soit exclue.

43

3. Molécule modifiée selon la revendication 2, dans laquelle l'acide aminé à ajouter au côté N-terminal du polypeptide isolé est une séquence d'acides aminés représentée par la formule (II), dans laquelle
Y1 représente Pro, Y2 représente Ser, Y3 représente une liaison simple,
Y1 représente un groupe amino, Y2 représente Ser, et Y3 représente Lys biotinylée, ou
Y1 représente Pro, Y2 représente Ser, et Y3 représente Lys biotinylée.

4. Molécule modifiée selon la revendication 2 ou 3, dans laquelle l'acide aminé à ajouter au côté C-terminal du polypeptide isolé est une séquence d'acides aminés représentée par la formule (I) suivante :

$$X1-X2-X3-X4 \quad (I)$$

dans laquelle

(1) X1 représente Lys biotinylée, X2 représente plusieurs acides aminés ou une liaison simple, X3 représente un acide aminé ou une liaison simple, et X4 représente un acide aminé ou un groupe carboxyle (à condition que lorsque X3 représente Pro, alors X4 représente un acide aminé),
(2) X1 représente un acide aminé à l'exception de Lys biotinylée ou une liaison simple, X2 représente plusieurs acides aminés ou une liaison simple, X3 représente Pro, et X4 représente un acide aminé, ou
(3) X1 représente un acide aminé à l'exception de Lys biotinylée ou une liaison simple, X2 représente plusieurs acides aminés ou une liaison simple, X3 représente un acide aminé à l'exception de Pro ou une liaison simple, et X4 représente β-Ala.

5. Molécule modifiée d'un polypeptide isolé, qui est dérivée d'un antigène spécifique d'une tumeur et se lie à une molécule du CMH de classe II, dans laquelle la modification est l'addition de plusieurs acides aminés au côté C-terminal et éventuellement au côté N-terminal du polypeptide isolé, dans laquelle l'acide aminé à ajouter au côté C-terminal du polypeptide isolé est une séquence d'acides aminés représentée par la formule (I) suivante :

$$X1-X2-X3-X4 \quad (I)$$

dans laquelle

(1) X1 représente Lys biotinylée, X2 représente plusieurs acides aminés ou une liaison simple, X3 représente un acide aminé ou une liaison simple, et X4 représente un acide aminé ou un groupe carboxyle (à condition que lorsque X3 représente Pro, alors X4 représente un acide aminé),
(2) X1 représente un acide aminé à l'exception de Lys biotinylée ou une liaison simple, X2 représente plusieurs acides aminés ou une liaison simple, X3 représente Pro, et X4 représente un acide aminé, ou
(3) X1 représente un acide aminé à l'exception de Lys biotinylée ou une liaison simple, X2 représente plusieurs acides aminés ou une liaison simple, X3 représente un acide aminé à l'exception de Pro ou une liaison simple, et X4 représente β-Ala.

6. Molécule modifiée selon la revendication 4 ou 5, dans laquelle l'acide aminé à ajouter au côté C-terminal du polypeptide isolé est une séquence d'acides aminés représentée par la formule (I), dans laquelle

(1) X1 représente Lys biotinylée, X2 représente un acide aminé ou une liaison simple, X3 représente un acide aminé ou une liaison simple, et X4 représente un acide aminé ou un groupe carboxyle (à condition que lorsque X3 représente Pro, alors X4 représente un acide aminé), ou
(2) X1 représente une liaison simple, X2 représente une liaison simple, X3 représente Pro, et X4 représente un acide aminé.

7. Molécule modifiée selon la revendication 6, dans laquelle l'acide aminé à ajouter au côté C-terminal du polypeptide isolé est une séquence d'acides aminés représentée par la formule (I), dans laquelle
X1 représente Lys biotinylée, X2 représente une liaison simple, X3 représente une liaison simple, et X4 représente Gly ou un groupe carboxyle,
X1 représente une liaison simple, X2 représente une liaison simple, X3 représente Pro, et X4 représente Ala ou β-Ala, ou

X1 représente Lys biotinylée, X2 représente une liaison simple, X3 représente Pro, et X4 représente β-Ala.

8. Molécule modifiée selon l'une quelconque des revendications précédentes, dans laquelle le polypeptide isolé comprend une séquence d'acides aminés dérivée de WT1, du PSA, de MAGE-3, de la survivine, du CEA, de la tyrosinase, du virus de l'hépatite C ou du virus EB, et se lie à une molécule du CMH de classe II.

9. Molécule modifiée selon la revendication 8, dans laquelle le polypeptide isolé comprend une séquence d'acides aminés dérivée de WT1 et se lie à une molécule du CMH de classe II.

10. Polypeptide isolé comprenant la séquence d'acides aminés représentée par SEQ ID NO : 37, SEQ ID NO : 38 ou SEQ ID NO : 40.

11. Agent antitumoral comprenant la molécule modifiée selon l'une quelconque des revendications 1 à 9, ou le polypeptide isolé selon la revendication 10.

12. Agent antitumoral selon la revendication 11, comprenant en outre un polypeptide isolé qui est dérivé d'un antigène spécifique d'une tumeur et se lie à une molécule du CMH de classe I.

13. Agent antitumoral selon la revendication 11 ou 12, comprenant en outre un adjuvant.

14. Agent antitumoral selon la revendication 13, dans lequel l'adjuvant est un vaccin pertussique.

15. Composition comprenant la molécule modifiée selon l'une quelconque des revendications 1 à 9 ou le polypeptide isolé selon la revendication 10, un polypeptide isolé qui est dérivé d'un antigène spécifique d'une tumeur et se lie à une molécule du CMH de classe I et un adjuvant, destinée à être utilisée dans le traitement d'une tumeur.

16. Procédé pour examiner si un sujet à tester a une molécule du CMH de classe II qui se lie à un polypeptide isolé contenu dans une molécule modifiée, comprenant la culture d'une population de cellules présentatrices d'antigène dérivée du sujet à tester et la molécule modifiée selon l'une quelconque des revendications 1 à 9, et la confirmation de la liaison de la population des cellules présentatrices antigène et de la molécule modifiée.

Fig. 1

Fig. 2

Fig. 3

Fig. 4

Fig. 5

Fig. 6

Fig. 7

Fig. 8

## Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

**Fig. 28**

Fig. 29

Fig. 30

Fig. 31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008096831 A
- EP 2423310 A
- WO 2010123065 A
- WO 0006602 A
- WO 0026249 A
- WO 2006030770 A
- WO 2005105993 A
- JP 2011273922 A

### Non-patent literature cited in the description

- **FALLARINO et al.** *J Immunol,* 2000, vol. 165, 5495-5501
- **JIN et al.** *Int J Cancer,* 2004, vol. 111, 558-567
- **NISHIMURA et al.** *J Exp Med,* 1999, vol. 190, 617-627
- **XIE et al.** *J Exp Med,* 2010, vol. 207, 651-667
- **KEENE ; FORMAN.** *J Exp Med,* 1982, vol. 155, 768-782
- **BENNETT et al.** *Nature,* 1998, vol. 393, 478-480
- **RIDGE et al.** *Nature,* 1998, vol. 393, 474-478
- **KENNEDY ; CELIS.** *J Immunol,* 2006, vol. 177, 2862-2872
- **MUELLER et al.** *J Immunol,* 2006, vol. 176, 7379-7384
- **JANSSEN et al.** *Nature,* 2003, 852-856
- **SHEDLOCK ; SHEN.** *Science,* 2003, vol. 300, 337-339
- **MARZO et al.** *J Immunol Methods,* 2000, vol. 165, 6047-6055
- **WONG et al.** *J Immunol,* 2008, 3112-3131
- **YAMAZAKI et al.** *Blood,* 2010, vol. 110, 4293-4302
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken (Basics and experiments of peptide synthesis). Maruzen Co, 1975
- **HARUAKI YAJIMA ; SHUNPEI SAKAKIBARA.** Seikagaku Jikken Koza (Biochemical Experiment) 1. *Tanpakushitsu no Kagaku (Chemistry of Proteins),* 1977, vol. IV, 205
- Zoku Iyakuhin no Kaihatsu (A sequel to Development of Pharmaceuticals). Peptide Synthesis. Hirokawa Shoten, vol. 14
- **MURPHY, K. et al.** *Science,* 1990, vol. 250, 1720-1723
- **SHIMONKEVITZ, R. et al.** *J Immunol,* 1984, vol. 133, 2067-2074
- **BUUS, S. et al.** *Science,* 1987, vol. 235, 1353-1358
- **SABATINI, R. et al.** *Anal Biochem,* 2007, vol. 363, 255-262
- **SHERMAN, L. et al.** *J Exp Med,* 1992, vol. 175, 1221-1226
- **KOZLOWSKI, S. et al.** *J Exp Med,* 1992, vol. 175, 1417-1422
- **BERSANETTI, P. et al.** *Biochemistry,* 2004, vol. 43, 15729-15736
- **VANHOOF, G. et al.** *FASEB J,* 1995, vol. 9, 736-744
- **YU, D. et al.** *FEBS J,* 2010, vol. 277, 1126-1144
- **VORONOV, S. et al.** *FEBS Lett,* 2002, vol. 522, 77-82
- **DALKAS, G. et al.** *J Pept Sci,* 2009, vol. 16, 91-97
- **COATES, D.** *Int J Biochem Cell Biol,* 2003, vol. 35, 769-773
- **LAPTEVA, N. et al.** *Biochem Biophys Res Commun,* 2002, vol. 296, 194-200
- **DANILOV, S. et al.** *Exp Hematol,* 2003, vol. 31, 1301-1309
- **ROBERTSON, J. et al.** *J Immunol,* 2000, vol. 164, 4706-4712